# EUROPEAN PATENT SPECIFICATION

(11) **EP 1 297 144 B1**
(45) Date of publication and mention of the grant of the patent: **24.04.2013**
(21) Application number: 01947666.2
(22) Date of filing: 29.06.2001
(51) Int. Cl.: C07K 14/395, C12N 1/19, C12N 15/62, C12P 1/02, C12N 15/81

(54) **RECOMBINANT SACCHAROMYCES CEREVISIAE EXPRESSING CHIMERIC GLUCOSE TRANSPORTERS**
REKOMBINANTE SACCHAROMYCES CEREVISIAE, DIE CHIMÄRE GLUCOSE TRANSPORTERPROTEINE EXPRIMIEREN
SACCHAROMYCES CEREVISIAE RECOMBINANTE EXPRIMANT DES TRANSPORTEURS DE GLUCOSE CHIMERIQUES

(30) Priority: 29.06.2000 SE 0002460; 17.11.2000 SE 0004241
(43) Date of publication of application: 02.04.2003
(73) Proprietor: Cereduce AB, 411 26 Goteborg (SE)
(72) Inventor: BILL, Roslyn, Solihull B93 9DG West Midlands (GB); BOLES, Eckhard, 64293 Darmstadt (DE); GUSTAFSSON, Lena, S-431 69 Mölndal (SE); HOHMANN, Stefan, S-443 32 Lerum (SE); LARSSON, Christer, S-431 64 Mölndal (SE); ELBING, Karin, 22651 Lund (SE)
(74) Representative: Jones, Elizabeth Louise
(86) International application number: PCT/GB2001/003079
(87) International publication number: WO 2002/000880

(56) References cited:
- EP-A- 0 785 275
- WO-A-00/14258
- SHERWOOD PETER W ET AL: "A glucose transporter chimera confers a dominant negative glucose starvation phenotype in Saccharomyces cerevisiae." GENETICS, vol. 155, no. 2, June 2000 (2000-06), pages 989-992, XP001031164 ISSN: 0016-6731
- BROWN CELESTE J ET AL: "Multiple duplications of yeast hexose transport genes in response to selection in a glucose-limited environment." MOLECULAR BIOLOGY AND EVOLUTION, vol. 15, no. 8, August 1998 (1998-08), pages 931-942, XP001031140 ISSN: 0737-4038
- NISHIZAWA KAZUHISA ET AL: "Substrate recognition domain of the Gal2 galactose transporter in yeast Saccharomyces cerevisiae as revealed by chimeric galactose-glucose transporters." JOURNAL OF BIOLOGICAL CHEMISTRY, vol. 270, no. 6, 1995, pages 2423-2426, XP002182850 ISSN: 0021-9258
- K. Elbing ET AL: "Role of Hexose Transport in Control of Glycolytic Flux in Saccharomyces cerevisiae", Applied and Environmental Microbiology, vol. 70, no. 9, 1 September 2004 (2004-09-01), pages 5323-5330, XP55040005, ISSN: 0099-2240, DOI: 10.1128/AEM.70.9.5323-5330.2004

## Description

The present invention relates to a yeast having modified saccharide, particularly hexose, transporting properties and its use.

The purpose of the invention is to obtain a yeast having modified saccharide, particularly hexose transporting properties while simultaneously avoiding production of alcohol, in particular ethanol under aerobic conditions and high saccharide, particularly hexose concentrations.

Yeast glycolysis, the pathway which converts sugar into pyruvate, has a massive capacity as has been documented by the fact that 50-70% of the yeast's cellular protein consists of glycolytic enzymes. Not surprisingly, this pathway is controlled in a very complex way and by different partially redundant mechanisms (Fraenkel, 1982, Carbohydrate metabolism, p. 1-37, in "The molecular biology of the yeast Saccharomyces cerevisiae: Metabolism and gene expression "Cold Spring Harbor Laboratory, Cold Spring Harbor, N.Y.; Gancedo and Serrano, 1989, Energy-yielding metabolism, p. 205-259, in "The Yeasts", Rose and Harrison (Eds.), Second ed, vol. 3. Academic Press; Zimmermann and Entian, 1997, Yeast sugar metabolism, in "Biochemistry, genetics, biotechnology and applications.", Technomic Publishing Co., Lancaster PA). Some of these seem to be shared with glycolytic pathways from other organisms up to humans and higher plants, but others appear to be unique to yeast.

Regulation of yeast glycolysis occurs via allosteric control of key enzymes in the pathway such as phosphofructokinase (PFK) and pyruvate kinase (Blazquez et al., 1993, FEBS Lett., 329, p51-54; Boles et al., 1996, Mol. Microbiol., 20, p65-76; Campbell-Burk and Shulman, 1987, Ann. Rev. Microbiol., 41, p595-616; Davies and Brindle, 1992, Biochemistry, 31, p4729-4735; Gancedo and Serrano, 1989, supra; Heinisch et al., 1996, J. Biol. Chem., 271, p15928-15933). For a long time PFK was considered to be the (single) rate-limiting step in glycolysis. The development of metabolic control analysis theory has shown this to be an oversimplification (Fell, 1997, in "Understanding the Control of Metabolism", Portland Press, London and Miami).

A control mechanism which is probably specific to yeast but of central importance, operates at the level of hexokinase and involves trehalose metabolism (Thevelein and Hohmann, 1995, Trends Biochem. Sci., 20, p3-10). Inactivation of trehalose-6-phosphate synthase causes an absence of trehalose accumulation, but also a growth defect, specifically when grown on glucose (González et al., 1992, Yeast, 8, p183-192; Van Aelst et al., 1993, Mol. Microbiol., 8, p927-943). The expression of heterologous trehalose-6-phosphate synthase in a mutant of *S. cerevisiae* lacking the same enzyme restored trehalose-6-phosphate levels as well as growth on glucose and glucose influx, at least partially (Bonini et al., 2000, Biochemical Journal, 15, p261-268).

Also other metabolites and/or co-metabolites such as ATP, ADP, NAD+ and Pi, may be involved at different levels such as allosteric control and the so-called "thermodynamic control" (alternatively "concentration" or "metabolite control") (Gancedo and Serrano, 1989, supra). A strong "negative" correlation between the ATP content and glycolytic flux has been found, which points at an allosteric control of flux. In a recent study it has been shown that the target for ATP inhibition in permeabilised cells was mainly at the level of phosphofructokinase and pyruvate kinase (Larsson et al., 2000, Yeast, 16, p797-809).

Altering expression of genes encoding glycolytic enzymes and proteolysis of glycolytic enzymes are other levels of regulation and control, which is far from understood when considering glycolytic control (Hohmann, 1997, p 187-211, in "Yeast sugar metabolism. Biochemistry, genetics, biotechnology and applications", Zimmermann and Entian (Eds.), Technomic Publishing Co. Inc., Lancaster, PA; Larsson et al., 1997, J. Bacteriol., 179, p7243-7250).

The yeast *S. cerevisiae* has a remarkable metabolic flexibility and it is one of the few yeasts which is able to grow fermentatively under strict anaerobic conditions (Visser et al., 1990, Appl. Environ. Microbiol., 56(12), p3785-3792). During aerobic conditions and with simultaneously relatively high external glucose concentrations, the Crabtree effect is exerted, which results in ethanol production also during aerobic growth on fermentable sugars (Fraenkel, 1982, supra; Gancedo and Serrano, 1989, supra). A number of causes of the Crabtree effect have been put forward. Firstly, it has been suggested that increased extracellular (or intracellular) levels of glucose cause repression of the activity of key elements of the respiratory pathway leading instead to processing via the fermentation pathway. Alternative explanations suggest that the effect may simply occur through overload of the glycolytic pathway leading to a shunt of carbon sources to fermentative processes.

As mentioned above, glucose repression (= (carbon) catabolite repression) may be part of the explanation for aerobic ethanol production during growth on fermentable sugars (Fraenkel, 1982, supra; Gancedo and Serrano, 1989, supra; Gancedo, 1992, Eur. J. Biochem., 206, p297-313). The primary effect of glucose repression is that glucose and fructose are the preferred carbon sources if a mixture of different sources is available. There is a whole series of genes involved in glucose repression and the extent of repression is correlated with the glucose uptake capacity. This suggests that the rate of glucose utilisation determines the strength of the relevant glucose signal (Gancedo, 1998, Microbiol. Mol. Biol. Rev., 62, p334-361).

However, it has been proposed that the extracellular (or intracellular) glucose concentration, rather than the glucose flux, triggers glucose repression (Meijer et al., 1998, J. Biol. Chem., 273, p24102-24107). If the glucose is sensed inside the cell then the transporters may have an indirect role not only in the generation of the initial signal but also in the maintenance of such a signal (Reifenberger et al., 1997, Eur. J. Biochem., 245, p324-333; Walsh et al., 1994, J. Bacteriol., 176, p953-958).

The intracellular glucose concentration has recently been shown to be much higher than reported previously, i.e. around 1.5 mM. This concentration is sufficient to reduce glucose influx by 50% (Teusink et al., 1998, J. Bacteriol., 180, p556-562). The authors of this study concluded that intracellular glucose is a strong candidate for regulation of glucose import and hence glycolysis.

During different external sugar concentrations, yeast cells exhibit high (Kₘ approx. 1-3 mM) or low (Kₘ approx. 10-50 mM) affinity sugar uptake systems (Bisson and Fraenkel, 1983, Proc. Natl. Acad. Sci. USA, 80, p1730-1734; Walsh *et al*., 1994, supra). Cells growing in low glucose media often display both a high affinity component and a low affinity component (Bisson and Fraenkel, 1983, supra). Glucose is transported into the yeast cell by facilitated diffusion through different specific carrier proteins (Bisson et al., 1993, Crit. Rev. Biochem. Mol. Biol., 28, p259-308).

Genetic studies have implicated a gene family, the *HXT*-family, including 20 homologous genes in encoding hexose transporters (Kruckeberg, 1996, Arch. Microbiol., 166, p283-292; Diderich et al., 1999, J. Biol. Chem., 274, p15350-15359; Ozcan and Johnston, 1999, Microbiol. Mol. Biol., 63, p554-569). The *HXT* family belongs to the major facilitator superfamily (Pao et al., 1998, Microbiol. Mol. Biol. Rev., 62, p1-34). Different members of the family are expressed to different levels depending on the external glucose concentration (Diderich *et al.,* 1999, supra). *HXT1* is induced by high glucose concentrations, whereas *HXT2, HXT4,* and *HXT6-7* are induced at low glucose concentrations and *HXT3* is induced irrespective of glucose concentration. Furthermore, transcription of *HXT1-7* is correlated to the extracellular glucose concentration. Also other conditions, such as nitrogen availability and aerobicity/anaerobicity affect the expression of members of the *HXT* family (Reifenberg et al., 1995, Mol. Microbiol., 16, p157-167; Reifenberger *et al*., 1997, supra; Diderich *et al.,* 1999, supra).

The *HXT* family also includes *GAL2* that encodes a galactose transporter, which also transports glucose, and *SNF3* and *RGT2,* encoding putative sensors of high and low glucose concentrations, respectively (Diderich *et al*., 1999, supra; Ozcan and Johnston, 1999, supra). The putative sensor proteins probably serve as glucose receptors and contains unusually long C-terminal tails that are predicted to be in the cytoplasm (Ozcan et al., 1996, Proc. Natl. Acad. Sci. USA, 93, p12428-12432; Ozcan et al., 1998, EMBO. J., 17, p2566-2573).

Modification of *Saccharomyces cerevisiae* by constructing DNA constructs which comprise a HXT-gene, in particular by using HXT1 and/or HXT3-genes, for increasing the production of ethanol, in particular for producing alcoholic beverages and liquor, is previously known from EP-A-0 785 275.

However, *Saccharomyces* yeast that produce little or no alcohol under aerobic conditions whilst still exhibiting growth, are not known.

For several thousands of years yeast has been used for the preparation of alcoholic beverages and in the future this process might become even more important for the production of ethanol as a renewable fuel. However, many more products other than alcohol can potentially be produced by yeast from renewable resources. For example other valuable substances such as fine chemicals, non-alcoholic drinks and homologous and/or heterologous compounds such as proteins or low molecular metabolites could, and in many cases, are produced.

In order to compete successfully with production processes based on other techniques, e.g. using fossil raw material (such as oil, which may be used as the basis for producing some products, e.g. fine chemicals), the highest possible yield is required. A potential problem is that "good" carbon sources such as glucose often cause repression of genes required for synthesis of the desired substance. As a result the yield will be low or the product may not even be formed. To overcome, or at least minimise, these effects different cultivation techniques such as fed-batch cultivation or growth in a chemostat is often employed, which avoids general increases in the concentration of the carbon source, e.g. glucose, which could lead to repression.

It has however now surprisingly been found that by appropriate modification of the saccharide transport capabilities of the yeast, processing through the fermentation pathway of *Saccharomyces cerevisiae* can be avoided, even in the presence of high concentrations of saccharide as the carbon source, e.g. fermentable sugars such as glucose, but with maintenance of good growth and carbon source consumption. No other workers in the field have produced yeast with such advantageous properties.

The present invention thus offers a solution to the existing problems of processing through the fermentative pathway under aerobic conditions and high saccharide levels since the yeasts containing the constructs of the invention seem to be relieved from the Crabtree effect, e.g. relieved from glucose repression. Hence, if fermentation is largely absent (e.g. via the absence of glucose repression), high yields of different substances can be obtained without resorting to sophisticated cultivation techniques.

The present invention thus relates to non-ethanol producing strains of *Saccharomyces cerevisiae* having specific hexose transporting properties, whereby changes in the hexose transporting gene provides one way of obtaining said non-ethanol producing property.

The invention described herein thus particularly relates to a chimeric construct between saccharide, e.g. glucose and/or galactose (preferably glucose), transporters, in particular between a high (for example HXT7) and a low (for example HXT1) affinity hexose transporter of the yeast *Saccharomyces,* preferably *S. cerevisiae.*

Apart from traditional processes such as baker's yeast production, the industrial applications also include production of homologous and/or heterologous substances such as proteins or low molecular metabolites, as well as bulk and fine chemicals, food stuffs including yeast extracts, functional food and therapeutic agents. Another application area is the production of fermented non-alcoholic beverages.

Thus the present invention provides a modified *Saccharomyces* yeast as defined below which produces lower levels of alcohol, in particular ethanol, than wild-type yeast under aerobic conditions and saccharide concentrations of 5mM or more, preferably 2% glucose, and which exhibits a growth rate of at least 30% of the wild-type yeast.

Alternatively stated, the present invention provides a *Saccharomyces cerevisiae* strain as defined below having modified hexose transporting properties, which is non-ethanol producing under aerobic growth and high hexose concentrations. Preferably said *Saccharomyces cerevisiae,* has heterologous and/or homologous expressed substances as described hereinafter.

Thus in a first aspect the present invention provides a modified *Saccharomyces cerevisiae* yeast which produces lower levels of ethanol than wild-type yeast under aerobic conditions and saccharide concentrations of 5mM or more and which exhibits a growth rate of at least 30% of the wild-type yeast, wherein said yeast contains a chimeric nucleotide sequence (a chimeric construct) which is stably transformed into the genetic material of the yeast, wherein the construct comprises a sequence having the form

A - B

wherein
A is a first sequence comprising nucleotide bases w to x;
B is a second sequence comprising nucleotide bases (x+y) to z;
wherein
A is from a nucleotide sequence encoding a low affinity saccharide transporter HXT-1 or HXT-3 of the yeast *Saccharomyces* or a sequence which hybridizes to the encoding sequence under non-stringent binding conditions of 6 x SSC/50% formamide at room temperature and washing under conditions of high stringency or a sequence which has at least 95% sequence identity to the encoding sequence or a sequence complementary to any of the aforesaid sequences
and
B is from a nucleotide sequence encoding a high affinity saccharide transporter HXT-2, 4, 6, 7 or GAL2P of the yeast *Saccharomyces* or a sequence which hybridizes to the encoding sequence under non-stringent binding conditions of 6 x SSC/50% formamide at room temperature and washing under conditions of high stringency or a sequence which has at least 95% sequence identity to the encoding sequence or a sequence complementary to any of the aforesaid sequences
and
the values w and x are first and second nucleotide positions within the nucleotide sequence of said low affinity saccharide transporter and (x+y) and z are first and second nucleotide positions within the nucleotide sequence of said high affinity saccharide transporter, and;
w is 1 to 50;
x is a number from 400 to 900;
y is less than 3; and
z is from 1600 to 1713,
wherein said yeast produces less than 50% ethanol compared to the wild-type yeast.

Said yeast produces less than 50%, e.g. less than 30, 20 or 10% alcohol (particularly ethanol), especially preferably less than 5%, e.g. less than 3, 2 or 1% alcohol (particularly ethanol) compared to wild-type. Conveniently, this may correspond to alcohol (preferably ethanol) levels less than 0.6g/l, e.g. less than 0.25g/l, especially preferably, less than or equal to 0.15g/l, e.g. less than or equal to 0.12g/l, under the conditions of assay.

Preferably, said yeast exhibits a generation time of at least 30%, 35, 40, 45, 50, 60, 70 or 80% (preferably at least 60, 70 or 80%) relative to wild-type yeast, e.g. a generation time of less than 4.5h, e.g. less than 3.9, 3.5 or 3.0h or an equivalent growth rate.

In a particularly preferred feature therefore, the present invention provides a modified *Saccharomyces* yeast as defined above which produces less than or equal to 0.15g/l alcohol (in particular ethanol) under aerobic conditions and saccharide concentrations of 5mM or more, preferably 2% glucose, and which exhibits a growth rate of more than 50% of the wild-type yeast.

The modified yeast of the invention may exhibit reduced glucose consumption, e.g. less than 50%, e.g. less than or equal to 40% less than wild-type yeast, e.g. less than 5mmol glucose/(g biomass.h), but preferably consumes more than 20%, e.g. more than 30, 40 or 50% compared to wild-type.

The growth yield of the yeasts of the invention is at least 30%, e.g. from 30 to 120%, e.g. more than 40 or 50%, preferably more than 60, 80 or 100% of the wild-type yeast, e.g. more than 0.20, e.g. more than 0.35, 0.40 or 0.50g biomass/g glucose.

Preferably the oxygen consumption of yeasts of the invention is improved relative to wild-type yeast, ie. is at least 100%, preferably at least 150, 175 or 200% relative to wild-type yeast, e.g. more than 2, 2.5, 3 or 4 mmol O₂/(g biomass.h).

Especially preferably the yeasts of the invention exhibit modified saccharide, e.g. hexose, e.g. glucose transporting properties. The transport of other saccharides, particularly galactose and/or maltose may also be affected.

Alteration of the saccharide transporting properties of yeast is conveniently achieved by introducing exogenous molecules related to naturally occurring transport molecules. Especially preferably chimeric constructs between portions of known transporter molecules may be generated and incorporated into yeast cells and thereby affect their trafficking of saccharides and their use of the respiratory and fermentation pathways under aerobic conditions.

The Hxt-family and other saccharide transporters have very similar topology and are proteins with 12 transmembrane domains. It has now been found that combining portions from different molecules provides functional transporter molecules which exhibit altered transporting properties. Without wishing to be bound by theory, it appears that such chimeras result in glucose derepression thus leading to enhanced use of the respiratory pathway and thus reduced alcohol production.

As described hereinafter in more detail, various chimeric molecules have been produced and integrated into the genome of *Saccharomyces cerevisiae.* In particular the development and testing of 2 modified yeast are described which contain chimeric constructs between portions of HXT1 and HXT7.

In the case of the first modified yeast (KOY.TM6 also referred to herein as KOY.TM6P), the fusion between the two genes has been effected in the region encoding transmembrane region 6 (TM6). This strain, KOY.TM6P, shows derepressed properties during growth on glucose in the presence of oxygen i.e. only minute amounts of ethanol are formed (less than 0.12g/l during growth on 2% Glucose). Simultaneously, a relatively high growth rate with a generation time of 3.8h, sugar consumption rate 3.1-3.9 mmol/(g biomass.h) and growth yield of 0.41g biomass/g glucose is obtained.

The second modified yeast, KOY.TM4 (also referred to herein as KOY.TM4P) contains a construct in which the genes have been attached in the region encoding transmembrane region 4. This modified yeast shows growth properties very similar to those seen for KOY.TM6. It has a growth rate with a generation time of 3 to 3.9h and a yield of 0.34 g biomass/g of glucose.

Thus, yeasts of the invention contain a chimeric nucleotide sequence (a chimeric construct), as defined hereinafter, which is stably transformed into the genetic material of the yeast, wherein the construct comprises 2 or more nucleic acid sequences encoding different saccharide, e.g. glucose and/or galactose (preferably glucose), transporters, ie. the construct comprises at least a first sequence from the nucleotide sequence encoding a first saccharide transporter (or a sequence related to or derived therefrom) and a second sequence from the nucleotide sequence encoding a different second saccharide transporter (or a sequence related to or derived therefrom), wherein said saccharide is preferably glucose.

The construct is between at least a high and a low affinity hexose transporter of the yeast *Saccharomyces,* preferably *Saccharomyces cerevisiae,* which form said first and second saccharide transporters as described above. The low affinity transporters are selected from HXT1 or HXT3, and the high affinity transporters are selected from HXT2, 4, 6, 7, or GAL2P, preferably from *Saccharomyces cerevisiae* or sequences related to or derived from the sequences of such molecules as defined herein. In particular, analogous and related sequences from other species or genera, e.g. from bacteria, plants or animals, may be used to produce the chimeras providing they have the required sequence identity or hybridizing properties.

A *Saccharomyces cerevisiae* comprising any combination of one or more of functional hexose transporters of the group HXT1 to HXT17, GAL2, SNF3, RGT2, AGT1, YDL247w and YJR160c may be generated. However, the invention concerns combinations of low and high affinity transporters as described hereinbefore. The amino acid sequences of such molecules are as described in P32465 (HXT1), P23585 (HXT2), P32466 (HXT3), P32467 (HXT4), P38695 (HXT5), P39003 (HXT6), P39004 (HXT7), S50771 (HXT8), S50708 (HXT9), S48313 (HXT10), S49600 (HXT11), S50356 (HXT12), S50520 (HXT13), S63299 (HXT14), S67809 (HXT15), S57187 (HXT16), S63405 (HXT17), P13181 (GAL2P), A31928 (SNF3), S67684 (RGT2), S64624 (AGT1), S67812 (YDL247w) and S57190 (YJR160c) in which the accession numbers prefixed with S or A refer to those from the PIR Database and those prefixed with P refer to those from the Swiss-Prot database.

Also included within the scope of the invention are chimeras made from one or more sequences related to or derived from the above described amino acid sequences (or chimeric constructs made from the nucleotide sequences encoding one or more sequences related to or derived from the above described sequences) as defined in the claims, e.g. one (or more) naturally occurring sequence may be combined with a sequence derived from a different naturally occurring molecule or a combination between 2 (or more) sequences each related to a different molecule may be used.

Especially preferably, the nucleotide sequence encoding a chimera of, or for use in, the invention, may comprise
one or more sequences or portions thereof (particularly as described hereinbelow) of the sequences encoding any one of HXT1, HXT2, HXT3, HXT4, HXT6, HXT7 and GAL2P, preferably from *Saccharomyces cerevisiae,* in chimeric nucleotide sequences as described above,
   or a sequence which hybridizes to said sequence or portion thereof under non-stringent binding conditions of 6 x SSC/50% formamide at room temperature and washing under conditions of high stringency, e.g. 2 x SSC, 65°C, where SSC = 0.15 M NaCl, 0.015M sodium citrate, pH 7.2,
   or a sequence which exhibits at least 95% e.g. at least 98% sequence identity to said sequence or portion thereof (as determined by, e.g. FASTA Search using GCG packages, with default values and a variable pamfactor, and gap creation penalty set at 12.0 and gap extension penalty set at 4.0 with a window of 6 nucleotides),
or a sequence complementary to any of the aforesaid sequences.

Such sequences or portions thereof may comprise a first or second sequence as described hereinbelow.

"Portions" as referred to above, preferably comprise at least 30% of the wild-type sequence, e.g. at least 50, 70 or 90% of the sequence, e.g. comprise 300 or more bases, preferably 500 or more or 600 or more bases. Portions as referred to in connection with amino acid sequences comprise comparable lengths as those encoded by the above described nucleotide sequences, e.g. 100 or more residues, preferably more than 180 or 200 residues.

Alternatively viewed, especially preferably the nucleotide sequences encoding a chimera of, or for use in, the invention, encodes an amino acid sequence which may comprise
one or more sequences or portions thereof (particularly as described hereinbelow) of the sequences of any one of HXT1, HXT2, HXT3, HXT4, HXT6, HXT7 and GAL2P, preferably from *Saccharomyces cerevisiae,* in chimeric amino acid sequences as described above,
   or a sequence which exhibits at least 95% e.g. at least 98% sequence identity to said sequence or portion thereof (as determined by, e.g. using the SWISS-PROT protein sequence databank using FASTA pep-cmp with a variable pamfactor, and gap creation penalty set at 12.0 and gap extension penalty set at 4.0, and a window of 2 amino acids).

Such sequences or portions thereof are encoded by a nucleotide sequences which may comprise a first or second sequence as described hereinbelow.

Preferably, where the nucleic acid sequences used to make the chimeras are related to or derived from the naturally-occurring sequences (and preferably fall within the above described families), the related or derived sequences encode a functionally equivalent protein or precursor or portion thereof.

Preferably therefore, the invention extends to a *Saccharomyces cerevisiae* as defined above, comprising the combination of one or more functional hexose transporters genes described herein, the DNA sequences being derived from these genes by substitution, deletion, or addition of one or more nucleotides in such a way that the DNA sequence still encodes a protein capable of transporting hexose(-s), e.g. glucose.

The invention also extends to chimeric constructs encoding proteins which are functionally equivalent to those described above and below, e.g. which are formed by combination of naturally-occurring molecules (or sequences related to or derived therefrom, e.g. functional equivalents), wherein that combination may be further modified, e.g. by substitution, deletion or addition, to provide a sequence encoding a functionally equivalent protein or precursor or portion thereof as defined hereinbelow, wherein preferably said functionally-equivalent protein satisfies the identity defined above, e.g. has at least 98% identity to the sequence formed by the combination, or wherein the encoding sequence satisfies the identity or hybridizing conditions described above.

Thus in a further feature the invention provides a modified recombinant *Saccharomyces cerevisiae* as described herein expressing a modified gene (i.e. a chimeric construct as described herein) deriving from the genes of the low and high saccharide transporters described herein, or derived transformants (e.g. functionally equivalent nucleic acid molecules) thereof falling within the definition described hereinbefore.

Thus the present invention provides a nucleotide sequence comprising a chimeric nucleotide sequence as described herein or a functionally equivalent nucleic acid molecule thereto, satisfying the identity and/or hybridizing conditions described above or a complementary sequence thereof, and modified yeast cells containing the same.

As described above, the chimeras are made between low affinity transporters HXT1 or HXT3 and high affinity transporters HXT2, 4, 6, 7 (especially preferably HXT6 or 7) or GAL2P.

Particularly preferably chimeric constructs are made between HXT1 and HXT7 or sequences related to or derived therefrom, and used to transform yeast to provide the yeast of the invention. Preferably N-terminal portions of HXT1 are conjugated with C-terminal portions of HXT7. The alternative form with HXT7 at the N-terminus and HXT1 at the C-terminus is also described but not included within the scope of the invention.

Thus, the nucleotide sequence encoding the chimera (ie. the chimeric construct) described herein comprises a sequence having the form:

A - B

wherein
A is a first component (ie. the first sequence as described hereinbefore) comprising nucleotide bases w to x;
B is a second component (ie. the second sequence as described hereinbefore) comprising nucleotide bases (x+y) to z;
wherein A and B are derived from distinct molecules, e.g. HXT1 and HXT7, ie. from the nucleotide sequences of different saccharide transporters or from sequences related to or derived therefrom as defined hereinbefore;
w is a first position within the nucleotide sequence from which said first component is derived (e.g. HXT1 or a comparable sequence as described above), which is less than x, is 1 to 50, especially preferably 1 to 10, e.g. 1;
x is a second position within the nucleotide sequence from which said first component is derived and is greater than w and is a number from 400 to 900, e.g. 500 to 850 (a number of 300 to 1250 is also disclosed but is not in accordance with the invention);
y is an integer, less than 3, e.g. 1;
x+y is a first position within the nucleotide sequence from which said second component is derived (e.g. HXT7 or a comparable sequence as described above);
and z is a second position within the nucleotide sequence from which said second component is derived and is greater than x+y, from 1600 to 1713, e.g. 1675 to 1713;
wherein said values refer to the position within the nucleotide sequence of said saccharide transporter, e.g. HXT1 or HXT7 or a comparable sequence thereof.

The transporter molecules of *Saccharomyces cerevisiae* comprise 12 transmembrane sequences. In a preferred feature, the point of junction between the 2 or more portions which together form the chimera falls within the sequence of the 3rd to 9th transmembrane stretches. Thus, preferably x is a position in the sequence encompassed by the third to ninth transmembrane stretches of HXT1 or HXT7 or a comparable sequence, especially preferably 525 to 800, for example 525-575 and/or 720-770 especially preferably 545 to 560 and/or 735 to 750 and y is preferably 1 and z is preferably as described above.

Preferably chimeras of the invention have a total of 12 transmembrane domains (e.g. w is less than 10 and x is from 1675 to 1713). This may be achieved by for example combining some of the 12 transmembrane domains (e.g. domains 1 to 3-9, or parts thereof) from a first transporter and the remainder (3-9 to 12, or parts thereof) from a second transporter. However, more or fewer transmembrane domains are also contemplated and linker groups may optionally appear between one or more of the transmembrane domains. Thus, preferably said chimeras of the invention comprise from 6 to 18 transmembrane domains, e.g. 10 to 14, which are derived from 2 or more transporter molecules as described hereinbefore.

Said chimeras described above may contain more than 2 components, e.g. multiple portions, e.g. 2 or more sequences of the nucleotide sequence of one or more transporter molecules or sequences from the nucleotide sequences of more than 2 transporters as described herein, in which case the portions are provided in parallel, preferably to retain the transmembrane structure, e.g. 10-14, preferably 12 transmembrane structure, optionally with one or more linker groups between said portions providing said chimeras have sequences as described hereinbefore. A linker group may also be inserted between A and B above (ie. y may be more than 1), providing this does not affect the functionality of the chimera.

Especially preferably x is 551 or 741, w and y = 1 and Z = 1713. Alternatively, x may be 668. Chimeric polypeptides encoded by such nucleic acid molecules are set forth in sequences 1 and 2 (SEQ. ID Nos. 17 and 18 in the Sequence Listing). Such sequences, sequences comprising them, functionally equivalent polypeptides (satisfying the identity requirements described above) and nucleic acid molecules containing a nucleotide sequence encoding the same, form preferred aspects of the invention. In a preferred feature, said encoding nucleic acid sequences are as set forth in sequences 3 and 4 (SEQ ID Nos. 19 or 20 in the Sequence Listing). Yeast cells expressing these forms also form preferred aspects of the invention. In particular, a further aspect of the invention provides a modified recombinant *Saccharomyces cerevisiae* as described above denoted *Saccharomyces cerevisiae KOY.TM4P* or KOY.TM6P or derived transformants thereof which fall within the above definitions, particularly having the deposition number DSM 13832 or DSM 13555, respectively.

Nucleic acid molecules comprising nucleotide sequences as described above, sequences encoding chimeric proteins as described above and complementary sequences thereto form further aspects of the invention. Proteins or polypeptides comprising a sequence encoded by such nucleic acid molecules form further aspects of the invention. In addition, the invention extends to functionally equivalent proteins as described hereinbefore, in addition to those in which the amino acids have been chemically modified, including by deglycosylation or glycosylation. In particular, these variant proteins may be prepared by post-synthesis/isolation modification of the substrate without affecting functionality, e.g. certain glycosylation, methylation etc. of particular residues. Non-standard amino acid may be used, such as α-aminobutyric acid, penicillamine, pyroglutamic acid or conformationally restricted analogs, e.g. such as Tic (to replace Phe), Aib (to replace Ala) or pipecolic acid (to replace Pro).

Thus in a preferred aspect the present invention provides a polypeptide comprising a sequence encoded by the nucleic acid molecule comprising the chimeric nucleotide sequence of the invention or a complementary sequence thereto wherein the polypeptide comprises a functional chimeric saccharide transporter. Another embodiment relates to a vector or host cell comprising the nucleic acid molecule described above.

Nucleic acid molecules according to the invention may be single or double stranded DNA, cDNA or RNA, preferably DNA and include degenerate, substantially identical and hybridizing sequences as described before. Ideally however genomic DNA or cDNA is employed.

Such exogenous molecules may be introduced into yeast cells by any appropriate means. Suitable transformation or transfection techniques are well described in the literature. The nucleic acid molecules described above may be operatively linked to an expression control sequence, or a recombinant DNA cloning vehicle or vector containing such a recombinant DNA molecule. In particular, appropriate nucleic acid molecules may be introduced into vectors for appropriate expression in the cell. Alternatively, the naked DNA molecule may be introduced directly into the cell. Conveniently, transformation of yeast cells as described herein is effected by the lithium acetate transformation method (Burke *et al*., 2000, CSHL Press, p103-105) or by freeze-thaw or spheroplast methods or other appropriate techniques (see e.g. Dohmen et al., 1991, Yeast, 7, p691-692).

Appropriate expression vectors include appropriate control sequences such as for example translational (e.g. start and stop codons, ribosomal binding sites) and transcriptional control elements (e.g. promoter-operator regions, termination stop sequences) linked in matching reading frame with the nucleic acid molecules of the invention. Appropriate vectors may include plasmids and viruses (including both bacteriophage and eukaryotic viruses). Suitable viral vectors include baculovirus and also adenovirus, adeno-associated virus, herpes and vaccinia/pox viruses. Many other viral vectors are described in the art.

A variety of techniques are known and may be used to introduce the vectors into cells for expression. Such vectors and eukaryotic or prokaryotic cells, particularly yeast cells, into which said vectors have been transfected form further aspects of the invention.

As mentioned above, nucleic acid molecules of the invention, or vectors comprising the same, may be introduced into *Saccharomyces* yeast cells, especially preferably *Saccharomyces cerevisiae.* Such cells may correspond to wild-type cells. However, conveniently, said cells may be modified (e.g. represent an altered recombinant form) even prior to inclusion of the nucleic acid material relating to the chimeric molecules.

Thus in a preferred embodiment, the phenotype of the wild-type cell is modified to produce a null strain in terms of saccharide, particularly glucose, transporting molecules, ie. to provide a null strain without or with severely limited saccharide transporting properties, e.g. exhibiting a glucose uptake preferably less than 2nmol glucose/min/mg biomass. This may be achieved by modification of the genotype to remove or alter the genes encoding one or more of HXT1-17, Gal2, St11 and 3 maltose transporters with glucose affinity and transport properties, namely AGT1, YDL247w/MPH2 and YJR160c/MPH3 or by altering the expression of one or more of those genes (e.g. by deleting one or more necessary transcription or translation factors) or by impairing the function of one or more of the expressed transporters. Conveniently, all of the above described genes, control systems affecting these genes or expressed proteins may be altered or impaired such that no active transporters are available for transport. However, alternatively a sub-set of transporters or their genes or expression thereof may be affected, preferably at least HXT1-4,6,7 and GAL2. These effects may be achieved as described above by for example altering the genes of the transporters, by affecting the transcription or translation of the genes, or by dominant negative mutation methodologies. The nucleic acid molecule encoding the chimera as described herein may thereafter be introduced into the null strain to provide a saccharide transporting phenotype.

Constructs and particularly modified *Saccharomyces* strains having the above described properties have great potential as tools for preparative processes in which metabolic pathways can be manipulated and undesirable side-products avoided. A yeast strain that does not produce any ethanol has been sought for a long time. The novel strains will therefore serve as invaluable tools in future efforts of characterization, e.g. to find an explanation for the repression mechanism during aerobic growth on glucose in *S. cerevisiae.* Further, a strain that does not produce any ethanol even when grown on high sugar concentrations during aerobic batch cultivation is also of considerable interest in terms of exploitation for industrial use.

Thus, *Saccharomyces* cells of the invention may be used in any processes in which the production of alcohol and other products produced during fermentation is undesirable. Such processes include for example the production of non-alcoholic high or low molecular metabolites, or low or high molecular weight compounds such as bulk and fine chemicals, food stuffs, functional food, therapeutic agents and non-alcoholic or low alcohol beverages.

These products may be produced by unmodified yeast of the invention, e.g. non-alcoholic or low alcohol beverages may be produced using appropriate saccharide containing carbon sources, e.g. fruit juices. In this way, low or non-alcoholic wines, beers or other drinks may be generated. Non-alcoholic beverages preferably have the alcohol levels defined herein. However, in a preferred feature, a beer is produced with alcohol levels of less than 1% alcohol w/v, especially preferably <0.5%, and similarly wine may be produced with alcohol levels of less than 10%, especially preferably less then 2.5 or 1% alcohol w/v.

Alternatively, such products may be produced by the introduction of exogenous genetic material which encodes a product of interest. Alternatively, exogenous genetic material may be used which influences the metabolic activity of the cell, thus resulting in the production of, or increased production of, a product of the yeast cell, by biasing the metabolic processes of the cell to produce (or increase production of) a desired product. In a further alternative, endogenous genetic material may be modified to influence the metabolic activity of the cell and thus favour production of a desired product.

The products of the above described genetic engineering may occur naturally in said yeast cells prior to genetic engineering, but are made to exhibit improved production after said engineering. In such cases the exogenous material may for example encode a protein involved in the metabolic process (either directly or indirectly), or an inhibitor of a catabolic process, or may provide regulatory control of the expression of such proteins, e.g. an inducible or constitutive promoter.

Modification of endogenous genetic material may be performed to affect a metabolic process, e.g. a sequence encoding a protein or an inhibitor or a sequence providing regulatory control may be modified to thereby effect the functions of that component.

The products of metabolic engineering may however not occur in wild-type yeast, and in which case said engineering may result in expression of one or more polypeptides which is the product, or which makes production of the desired product possible.

In a preferred aspect however, the product is an entity capable of being produced by said yeast cell (prior to inclusion of constructs of the invention) but which is produced in increased quantities after the introduction of exogenous genetic material, or modification of endogenous genetic material, as described above. Thus, in a preferred feature, products which may be produced by yeast of the invention are amino acids, peptides, polypeptides, sugars, small polyols and carbon dioxide, to mention but a few.

Thus, exogenous or endogenous genetic material, encoding a product of interest or part thereof, or a polypeptide which directly or indirectly facilitates production of a product of interest, or exogenous or endogenous genetic material influencing the expression of the same, may be inserted into, or modified in, yeast cells of the invention, e.g. optionally by stable transformation into the cells' genome in the case of exogenous genetic material. The cells may then be grown under high saccharide concentrations under aerobic conditions and the product thus formed harvested.

Thus, in a yet further aspect the present invention provides a method of inserting exogenous material into a yeast cell as described above, to produce a yeast cell of the invention containing exogenous genetic material, said method comprising at least the steps of introducing said genetic material, preferably contained within a vector, into said cell, e.g. by transformation. Preferably the exogenous material encodes, or controls the expression of, a product which is, or which enables the production of, a product. Alternatively stated the exogenous material encodes said product or portion thereof or encodes a polypeptide (or part thereof) which facilitates the direct or indirect production of said product or portion thereof, or affects the expression of said product or polypeptide. Such a product may be a high or low molecular metabolite, or a low or high molecular weight compound such as a bulk or fine chemical, a food stuff, a functional food or a therapeutic agent, e.g. as described above. Cells obtainable by this method form further aspects of the invention.

These further modified yeast may then be used to produce the desired product which can then be isolated from the yeast cells. Thus in a further aspect the present invention provides the use of a *Saccharomyces cerevisiae* having modified hexose transporting properties and which is non-ethanol producing under aerobic conditions and high hexose concentrations, in the preparation and manufacture of substances including low or high molecular weight metabolites, preferably in the preparation and manufacture of bulk and fine chemicals, food stuffs, functional food, therapeutic agents or non-alcoholic beverages.

Alternatively viewed the invention further provides a method of preparing a product, e.g. a polypeptide, amino acid, sugar or polyol in a yeast of the invention containing the genetic material as described above, said method comprising growing said yeast cells under aerobic conditions in the presence of high saccharide, preferably glucose, concentrations. In an alternative preferred feature, as mentioned above, products are produced by yeasts of the invention which have not been further modified, but which produce low levels of alcohol and thus when grown on an appropriate medium produce products of interest, e.g. low or non-alcoholic beverages. The products thus produced as described above may be isolated.

In determining the growth, alcohol production, saccharide consumption or other parameters described herein the following test conditions are used. These parameters are assessed during the period of exponential growth under aerobic (O₂ non-limiting) conditions at 30°C, in the medium used by Verduyn et al. (1992, Yeast, 8, p501-517) at pH 5.00 in the presence of 2% glucose, with a starting culture of 0.3 to 0.7g dry biomass/l (preferably 0.3) in 1.51 fermentors, stirred at 1500rpm and an air inflow of 0.5 volume of air per vessel volume per minute (vvm).

Biomass content (dry weight) is determined as follows: 5 ml is removed from the culture and centrifuged for 5 minutes in pre-weighed dry weight tubes at sufficient rpm to pellet substantially all the cells. The pellet is washed once with 5 ml 0.9% NaCl and re-pelleted. The pellet is dried for 24 hours at 110°C before temperature equilibration and weighing.

Continuous gas analysis is effected using a carbon dioxide and oxygen monitor type 1308, Bruel and Kjaer, Naerum, Denmark, e.g. to calculate oxygen consumption rates.

Glucose and ethanol determinations are made in the samples as follows: 1.5 ml samples of the culture are centrifuged at 15000g for 1 minute and the resulting supernatants frozen at -20°C until analysis. Concentrations are determined using enzyme combination kits from Boehringer Mannheim, GmbH, Germany.

As used herein, a "modified" yeast refers to one which has been derived from or is related to a wild-type yeast but exhibits phenotypic and preferably also genotypic variation thereto. Preferably the genotype has been modified, particularly in relation to genes encoding saccharide transporting molecules, which results in an altered phenotype. Conveniently this may be achieved by recombination to remove and/or modify existing genetic information and/or alter and/or integrate exogenous genetic sequences. Preferably the modification relates to at least the inclusion of chimeric nucleic acid molecules as described herein. Additional modifications may however also be contemplated, preferably those which further affect the saccharide transporting molecules, e.g. by the creation of yeast cells which are unable to transport glucose or exhibit a severely restricted capacity to do so, prior to insertion of the chimera. These recombinant organisms form preferred features of the invention. Furthermore, additional genetic modifications may be contemplated, e.g. to introduce genetic material encoding a desirable product or portion thereof.

As used herein "wild-type" refers to the yeast strain from which the modified yeast is originally derived or to which it is related. When reference is made to % alcohol, growth etc. of yeast of the invention relative to wild-type, said wild-type refers to the unmodified, naturally occurring, yeast, prior to insertion of constructs of the invention or modification of the genome in any way, ie. does not refer to for example a null strain into which the construct is inserted, but rather refers to the naturally-occurring yeast from which the null strain is generated. Thus when the invention relates to modified *Saccharomyces cerevisiae* yeast, the wild-type is the *Saccharomyces cerevisiae* strain that has been modified. Wild-type sequences refer to those occurring in wild-type microorganisms such as those defined hereinbefore.

*"Saccharomyces"* refers to the genus of the wild-type yeast which is modified. *"Saccharomyces cerevisiae"* refers to the particular species which is preferably modified, and includes all strains falling within that particular species.

As used herein, "alcohol" refers to the production of alcohols such as glycerol and ethanol. However, in the tests described herein, the level of ethanol is tested. Glycerol contributes only a small proportion of the total alcohol content relative to ethanol. "Non-ethanol producing" refers to yeast producing the amounts of ethanol described above, e.g. less than or equal to 0.12g/l, under the conditions of assay.

The g/l levels of alcohol, in particular ethanol, refers to the amount produced per litre of culture under the test conditions when assessed continually at discrete time points. Maximum levels are generally achieved just prior to glucose depletion during batch cultivation.

"Aerobic conditions" refers to culture conditions which are not limiting for oxygen and thus allow use of respiratory pathways. "Aerobic growth" refers to growth, e.g. as measured by increasing optical density at 610nm and increase in biomass content under such conditions.

A "transporter" refers to a molecule responsible for transfer of the molecule to be transported from the extracellular culture medium into the cell or *vice versa,* i.e. effecting its passage, e.g. diffusion, across the plasma membrane. Chimeras may be produced using saccharide receptors such as SNF3 and RGT2 and sequences related to or derived therefrom but do not fall within the scope of the definition of transporters insofar as it relates to transporters which may be used to make chimeras of the invention.

"Hexose" or "saccharide" transporting properties refers to the ability of the modified yeast of the invention to take up saccharide from the extracellular culture medium.

A "functional hexose transporter" refers to a transporter molecule which may be a naturally occurring molecule or a functionally equivalent variant as described herein, which is able to transport a saccharide as described above. In reference to functional hexose transporters which may be used to make chimeras of the invention, in line with the definition of transporters, such functional hexose transporters also extend to molecules which have the function of saccharide receptors.

By "hexose transporter gene" is meant a gene encoding a protein which effects the passage, e.g. diffusion, of hexoses across the plasma membrane, for example the HXT4, HXT5, or HXT6 genes.

"Saccharide" refers to mono-, di- or polysaccharides which may be used as a carbon source by wild-type or modified *Saccharomyces* yeast of the invention. Preferably the saccharides are monosaccharides, particularly preferably glucose, fructose, mannose or galactose, or disaccharides such as maltose. Preferably such saccharides are in the naturally occurring D form. For use under test conditions, naturally occurring underivatized D form saccharides, such as D-glucose, are employed. "Hexose" refers to monosaccharides such as those mentioned above, e.g. glucose, fructose and galactose.

"Growth rate" as referred to herein is mathematically related to generation time and has the relationship: µ = ln(2)/G; wherein µ is the specific growth rate, and G is the generation time. As such, values for % generation time rate relative to wild-type yeast similarly provide preferred % growth rates.

"Generation time" is measured in hours and refers to the doubling time, ie. the time taken for each generation to be produced during culture, wherein the generations are assessed by reference to increases in the dry weight of the biomass. Other experiments have been performed which determine generation time by reference to readings at OD₆₁₀. In such cases TM6 yields a value of 2.9 and TM4 a value of 2.5. Definitions provided in the text however refer to the values determined using the dry weight measurement described above.

The "glucose consumption rate", refers to the amount of glucose consumed from the external medium during the test conditions, and is thus assessed by measurement of the glucose in the medium at at least 2 time points and calculation of the glucose consumed in the time between those time points. This value is provided in mmol glucose/(g biomass.h), wherein the g biomass refers to the dry mass of yeast in the culture.

"Biomass" refers to the mass attributed to the dry mass of the yeast in the culture.

The term "high hexose" concentrations used in the context of growing the *Saccharomyces cerevisiae* refers to any hexose concentration exceeding 1mM, preferably exceeding 5 mM present in the media, especially preferably more than 50mM, e.g. equal to or more than 1 or 2% hexose, preferably glucose. In the test conditions however a value of 2% glucose, ie. 111mM is used. Similar definitions apply to high saccharide or glucose concentrations. Products as described herein may be produced at various high levels of saccharide, e.g. more than (or equal to) 1, 2, 5, 10 or 20% saccharide. In particular, the higher saccharide levels may be used when producing the beverages described herein.

As used herein, "exponential growth" refers to an exponential rate of doubling the biomass.

As used herein a "chimera" refers to a nucleic acid molecule or polypeptide comprised of two or more sequences (component sequences) derived from two or more different molecules, preferably two sequences each derived from a different molecule. Thus such chimeras provide the result of a combination of such sequences. As described hereinbefore said component sequences (or the parent sequences of which they are a portion) may be naturally occurring or may be related to or derived from such naturally occurring sequences. Whilst conveniently the separate component sequences may be combined together by linking the respective sequences to one another, alternatively, particularly if the sequences are highly homologous, alternative techniques may be used to obtain the chimera, e.g. by mutation of appropriate sites within a particular molecule to arrive at a sequence which in effect reflects a chimera between two distinct sequences.

A "chimeric construct" refers to a nucleic acid molecule comprising at least a nucleotide sequence encoding or providing a chimera as defined above, optionally additionally comprising flanking sequences optionally containing functional elements, e.g. promoter sequences.

"Stably transformed" refers to the inclusion (preferably insertion into the genome) of the construct of the invention into yeast cells as described herein, such that the encoding region of said construct may be expressed, and the construct or part thereof encoding a chimera of the invention is present in the progeny of the yeast cell.

As used herein, "low affinity" transporters refer to those which bind glucose with a Kₘ of more than 20mM, e.g. from 50-100mM under the conditions described herein in Example 1. "High affinity" transporters refer to those having a Km of less than 20mM, preferably less than 10mM, e.g. 1-2mM.

Sequences which are "related to or derived" from nucleotide or amino acid sequences described herein refer to sequences which have been modified relative to those sequences, e.g. by substitution, deletion or addition. Preferably such sequences are functional equivalents as described herein. The sequences have the stated identity or hybridizing properties as described hereinbefore.

"Functionally-equivalent" proteins as used herein refers to proteins related to or derived from the native or naturally-occurring protein, where the amino acid sequence has been modified by single or multiple amino acid substitution, addition and/or deletion, but which nonetheless retain the same function, ie. are capable of transporting (or when used to make chimeras act as a receptor for) one or more saccharide molecules, e.g. glucose, to a lesser or greater extent that the naturally occurring molecules. Such proteins are encoded by "functionally-equivalent nucleic acid molecules" which are generated by appropriate substitution, addition and/or deletion of one or more bases. The functionally-equivalent protein or nucleic acid molecule satisfies the identity and/or hybridizing conditions as set forth hereinbefore.

Within the meaning of "addition" variants are included amino and/or carboxy terminal fusion proteins or polypeptides, comprising an additional protein or polypeptide fused to the chimeric portion.

Such functionally-equivalent variants mentioned above include natural biological variations (e.g. allelic variants or geographical variations within a species or alternatively in different genera, e.g. plants, animals or bacteria) and derivatives prepared using known techniques. For example, nucleic acid molecules encoding functionally-equivalent proteins may be produced by chemical synthesis or in recombinant form using the known techniques of site-directed mutagenesis including deletion, random mutagenesis, or enzymatic cleavage and/or ligation of nucleic acids. In particular nucleic acid molecules encoding functionally equivalent protein variants for the production of chimeras in accordance with the invention extend to analogues in different genera or species than the specific molecules mentioned herein.

"Precursors" of the naturally occurring proteins may be larger proteins which would be processed, e.g. by proteolysis to yield the substrate. Such precursors may take the form of zymogens, ie. inactive precursors of enzymes, activated by proteolytic cleavage.

"Portions" of functionally equivalent proteins are as described above, and do not themselves necessarily exhibit the activity of the parent molecule. These portions satisfy the identity (relative to a comparable region) or hybridizing conditions mentioned herein. Portions of products refers to portions which when combined with other entities form the desired product.

"A polypeptide or part thereof which facilitates the direct or indirect production of a product or portion thereof" refers to a molecule, or a part of a molecule or complex, which on expression in a yeast cell of the invention results in the production of the product or part therefore, e.g. by altering or contributing to pathways which affect its production.

A polypeptide "affects the expression" of a product or polypeptide when it exerts a regulatory control on said expression.

The following Examples are given by way of illustration only in which the Figures referred to are as follows:
Figure 1 shows oxygen consumption and carbon dioxide production under different phases of the growth cycle of *S. cerevisiae* of the prototropic KOY.PK2-1C83 wild-type strain;
Figure 2 shows oxygen consumption and carbon dioxide production under different phases of the growth cycle of *S. cerevisiae* of the prototropic TM6-expressing strain;
Figure 3 shows a semilog-plot of the optical density versus time giving the measure of biomass growth during aerobic growth on glucose by the TM6-expressing strain;
Figure 4 shows a continuous on-line plot of the ratio between the carbon dioxide production rate and the oxygen consumption resulting in continuous monitoring of RQ during aerobic growth on glucose of the TM6-expressing strain. Measurements during the first 20 hrs should not be considered because of limiting sensitivity;
Figure 5 shows on-line heat-production measurement for KOY.TM4P;
Figure 6 shows a semilog-plot of the optical density versus time giving the measure of biomass growth on glucose by the TM4-expressing strain; and
Figure 7 shows a semilog-plot of the biomass(g/l)/time(h) for A) TM6 and B) TM4.

### Example 1: Production of modified Saccharomyces cerevisiae strains containing chimeras of HXT1 and HXT7

The yeast HXT-family has clear similarities to the mammalian Glut1. Since this protein has 12 transmembrane domains with N- and C-terminus located in the cytoplasm (Mueckler, 1994, Eur. J. Biochem., 291, p713-725) it is very likely that yeast hexose transporters have similar topology in which the N and C termini are intracellularly located and the sequence joining the termini passes back and forth through the membrane to provide 12 transmembrane regions linked together by intracellular or extracellular loops of various lengths. Chimeras were produced which contained HXT1 at the N-terminus fused together with HXT7 in the transmembrane region. For example, KOY.TM6P (present innovation) which is fused in transmembrane region 6 is identical to HXT1 from the N-terminus up to the middle of transmembrane region 6 while the rest of the protein is equivalent to HXT7.

This gives in total 12 constructs named TMl to TM12 which have all been integrated into the genome.

### Materials and methods

### Strains

A CEN.PK strain with deletions for the genes of the sugar transporters *HXT1-7* has been shown to still grow on glucose (Wieczorke et al., 1999, FEBS Lett., 464, p123-128). Because of this the additional *HXT* genes (altogether 17 genes), as well as further genes (see below) have been deleted in order to obtain a true *null* strain in terms of glucose transport (Wieczorke *et al.,* 1999, supra) - EBY.VW4000. Although the physiological roles *of HXT8-17* have not been described so far (except that *HXT9* and *11* have been described to be involved in Multi-Drug-Resistance), they are certainly not silent during all conditions. A hxt5 deletion mutant does not show a clear phenotype in glucose media. It is expressed in glucose deprived cells, probably in order to ensure rapid utilisation of the sugar when it becomes available. The complete null strain is useful, since re-introduction of one or several specific hexose transporter from yeast or even from other organisms can be used to study up-take kinetics and sugar consumption kinetics of specific transporters. Hence, the null strain is in the first place a tool to study the properties of specific yeast or heterologous hexose

The wild-type strain CEN.PK2-1C, has the following genetic markers *Mata leu2-52, 112ura3-52, trpl-289, his3-*Δ*1, MAL2-8c SUC2.* This strain was made prototropic (KOY.PK2-1C83) by introducing the marker genes one by one by transformation. The "null mutant" EBY.VW4003 (*Mata leu2-52, 112ura3-52, trp1-289, his3-*Δ*1, M*Δ*L2-8c SUC2 Hxt1-17*Δ, *Gal2*Δ, *Stl1*Δ, 3 deleted maltose transporters with glucose affinity, namely AGT1, YDL247w and YJR160c; genomic cassette Pro-*HXT7::URA3*) is unable to grow on any concentration of glucose. The deletions in the strain to provide the "null strain", which is deleted in all known hexose transporters and putative transporters, was constructed using the lox P/Cre recombinase system (Gueldener et al., 1996, Nucl. Acids Res., 24, p2519). The strain was made prototropic (KOY.VW100) by transformation.

### Chimeras

Chimeras between HXTI and HXT7 were made using the PCR based method "in vitro overlap-extension" (Higushi et al., 1981, Nucl. Acids Res., 16, p7351-7367; Ho et al., 1989, Gene, 71, p51-59). The parts to be fused together were first amplified separately but with ends that can anneal with each other. In the second PCR step, flanking primers were used to only allow amplification when annealing of the two separate products had taken place.

For the preparation of TM4, the HXT1 part of TM4 was amplified from plasmid pTHXT1-2 (Reifenberger *et al*., 1997, supra describes the production of pTHXT1-1 in which the HindIII insert is in the reverse orientation compared to pTHXT1-2) using the following primers:
Forward primer: 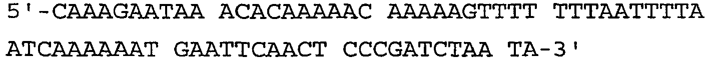
Reverse primer:
   5'-GGAGATAAAA CGGCAATACC ACCGACACCT AAACCA-3'

The HXT7 part of TM4 was amplified from plasmid P21 (Reifenberger et al., 1995, Mol. Microbiol., 16, p157-167) using:
Forward primer:
   5'-TGGTTTAGGT GTCGGTGGTA TTGCCGTTTT ATCTCC-3'
Reverse primer:
   5'-TTTGTAGACG TGGGTCTGCA GGCA-3'

For the preparation of TM6, the HXT1 part of TM6 was amplified from plasmid pTHXT1-2 using the following primers:
Forward primer: 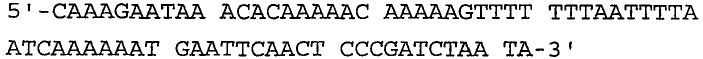
Reverse primer:
   5'-CTGGAACAAA TGTCATACCA CCAATCATAA ATAAGGCCCA G-3'

The HXT7 part of TM6 was amplified from plasmid P21 using:
Forward primer:
   5'-CTGGGCCTTA TTTATGATTG GTGGTATGAC ATTTGTTCCA G-3'
Reverse primer:
   5'-TTTGTAGACG TGGGTCTGCA GGCA-3'

The products from the first two separate reactions were mixed and a PCR run in which the forward primer from the HXT1 reaction and the reverse primer from the HXT7 reaction was used. Since the products from the two first reactions are made in such a way that they have overlapping regions, one obtains a product in which the HXT1 and HXT7 parts are fused together.

The construct KOY.TM6P was made by fusing HXT1 and HXT7 in the sixth trans membrane region. The N-terminus consists of bp 1-741 of HXT1 and the C-terminus of 742-1713 of HXT7. The construct TM4 was made by the same method used for the TM6 construct but the two genes were fused in TM-region 4. For TM4 this is bp 1-551 of HXT1 and 552-1713 of HXT7.

These constructs were introduced into the KOY.VW100 strain, which was made prototrophic by introducing URA3 prior to said introduction. The cassette used in the null-mutant is located in the former *HXT3-6-7* gene cluster and contains the constitutive promoter of *HXT7::K. lactis URA3::HXT7* terminator and was prepared as follows.

*Construction of the genomic expression cassette Sequence No. 5* (SEQ ID No.: 21 in the Sequence Listing):
A 392 bp *HXT7* promoter fragment was integrated into the genome of strain EBY.VW4000 (Wieczorke *et al.,* 1999, supra) into the former *HXT3-6-7* gene cluster region by using a modification of the PCR targeting technique, resulting in a very strong and constitutive *HXT7* promoter-terminator expression cassette (see Hauf et al., 2000, Enzyme Microb. Technol., 26, p688-698). Part of the *HXT7* promoter together with part of the *HXT7* coding region from -392 to +30 was amplified by PCR with primers PROHXT7-1 and PROHXT7-2 (see below), and plasmid p21-PST (Reifenberger *et al.,* 1995, supra) as a template. The PCR product was cleaved with SpeI at both ends and cloned in the correct orientation into the SpeI site of plasmid pUG6 (Gueldener *et al.,* 1996, supra) behind the second *loxP* site, resulting in plasmid pUG6-kPHXT7. This plasmid was then used as a template to generate by PCR with primers INTPH7-1 and INTPH7-2 a DNA molecule consisting of a *kanMX-HXT7p* marker cassette flanked by short homology sequences to the *HXT3* promoter (-770 to -720) and *HXT7* terminator regions. The 2.4 kb PCR product was transformed into strain EBY.VW4000 (whose *HXT3-6-7* gene cluster is replaced by a *loxP* site), selecting for resistance to G418 (200 mg liter⁻¹) on YPMaltose agar plates, and used to replace the *HXT3 promoter-loxP* region by the *kanMX-HXT7p* cassette. After transformation with plasmid pSH47, the *kanMX* marker was removed as described (Gueldener et al., 1996, supra), resulting in strain EBY.VW4002.

The *HXT7* gene was amplified by PCR from plasmid p21-PST with primers C1-IHXT7 and C4-IHXT7. The PCR product containing short homology sequences to the *HXT7* promoter-terminator expression cassette of strain EBY.VW4002 was transformed into this strain, selecting for growth on YPGlucose agar plates. Integration into the genomic expression cassette by homologous recombination resulted in strain JBY02 (*HXT7*+). To construct an FOA(5-fluoroorotic acid)-counterselectable marker-expression cassette in strain EBY.VW4002, the *K. lactis URA3* ORF was PCR-amplified from strain MS7-62 (gift of C. Falcone, Rome) with primers I-KURA31 and I-KURA32 (see below), resulting in a DNA-fragment with the *K1URA3* ORF flanked by short homology regions to the *HXT7* promoter and *HXT7* terminator. The DNA fragment was transformed into strain JBY02 selecting for growth on a synthetic medium without uracil and with maltose as the carbon source, resulting in strain EBY.VW4003 containing a genomically integrated *HXT7 promoter^{(-392 - +1)} - KlURA3 - HXT7 terminator* expression cassette. This strain was made prototropic (KOY.VW100) by transformation.
PROHXT7:
   5'-GGACTAGTGA TATCTCTCGT AGGAACAATTTCGG-3'
PROHXT7-2:
   5'-GGACTAGTTG CTCTGCAATAGCAGCGTC-3'
INTPH7-1: 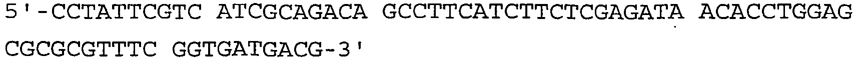
INTPH7-2: 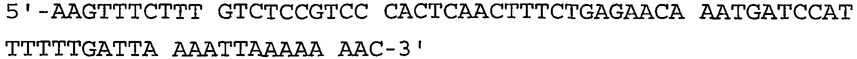
C1-IHXT7:
   5'-CCTGCGTGTT CTTCTGAGGTTC-3'
C4-IHXT7:
   5'-TTTGTAGACG TGGGTCTGCAGGCA-3'
I-KURA31: 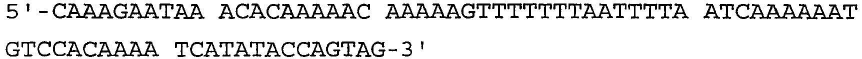
I-KURA32:

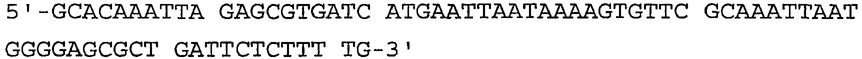

Integration of the different genes or constructs was done using homologous recombination. Recombinations were selected on YPGlucose (YPD 2% glucose) plates and then replica plated on to YNB 2% Maltose 5-FOA plates to select for out-recombination of URA3. To make the strains prototropic the URA3 was reintroduced by transformation and homologous recombination.

The chimeras or wild-type genes that have been integrated in the strain constitute the only hexose transporter.

The prototropic strains used in these experiments are wild type KOY.PK2-1C83, "null mutant" KOY.VW100, KOY.VWTM6P, KOY.VW101P and KOY.VW102P.

The strains used and generated are summarised in the following table:

| Strain | Genotype | Source |
|---|---|---|
| KOY.PK2-1C83 | MATa MAL2-8c SUC2 | Auxotropic: |
| | | K-D Entian (Van Dijken et al., 2000, Enzyme Microb. Technol., 26, p706-714 |
| | | Prototropic: This study |
| | | |
| KOY.VW100 | MATa MAL2-8c SUC2 hxt17ΔA | Auxotropic: Eckhard |
| | 112ura3-52 gal2Δ: :loxP stl1Δ: :loxP | |
| | agt1Δ: :loxP ydl247wΔ: :loxP | Boles (Wieczorke *et al.,* 1999, supra) and this study |
| | yjr160cΔ: :loxP hxt13Δ: :loxP | |
| | hxt15Δ: :loxP hxt16Δ: :loxP | |
| | hxt14Δ: :loxP hxt12Δ: :loxP | |
| | hxt9Δ: :loxP hxt11Δ: :loxP | Prototropic: This study |
| | hxt10Δ: :loXP hxt8Δ: :loxP | |
| | hxt514Δ: :loxP hxt2Δ: :loxP | |
| | Hxt367Δ: :loxP | |
| | Integration cassette: (located at the former site of HXT367) strong constitutive promoter from HXT7 the K. *lactis* URA3 ORF for 5-FOA counter selection and HXT7 terminator. | |
| KOY.TM4 | KOY.VW100 with TM4 construct integrated into cassette with subsequent out-recombination of *K. lactis* URA3 in the cassette. URA3 reintroduced to obtain a prototropic strain | This study |
| KOY.TM6P | KOY.VW100 with TM6 construct integrated into cassette with subsequent out-recombination of *K. lactis* URA3 in the cassette. | This study |
| | URA3 reintroduced to obtain a prototropic strain | |
| KOY.VW101P | KOY.VW100 HXT1 construct integrated into cassette with subsequent out-recombination of *K. lactis* URA3 in the cassette. | This study |
| | URA3 reintroduced to obtain a prototropic strain | |
| KOY.VW102P | KOY.VW100 HXT7 construct integrated into cassette with subsequent out-recombination of *K. lactis* URA3 in the cassette. | This study |
| | URA3 reintroduced to obtain a prototropic strain | |

The protein sequence of TM6 is as follows - Sequence No. 1 (SEQ. ID NO. 17 in the Sequence Listing):

At position 279 above, TM6 contains a tyrosine residue (shown in bold). This represents a modification relative to the naturally occurring sequence in HXT1 in which the residue is serine. As such, modification at this residue or comparable residues in other transporters forms a further preferred feature of the invention. However, sequences in which position 279 is a serine residue in the above sequence is also included within the scope of this invention.

The protein sequence of TM4 is as follows - Sequence No. 2 (SEQ. ID NO. 18 in the Sequence Listing):

Functional constructs which have been identified are TM 1,2,3,4, 5, 6,10,11,12. These constructs, which form preferred aspects of the invention, are made using the following portion of HXT1: 1-231 (TM1), 1-391 (TM2), 1-449 (TM3), 1-551 (TM4), 1-627 (TM5), 1-741 (TM6), 1-1010 (TM7), 1-1108 (TM8), 1-1214 (TM9), 1-1293 (TM10), 1-1438 (TM11), 1-1504 (TM12), with the remainder made up of HXT7, e.g. 232-1713 in the case of TM1. No transformants for TM7,8,9 have yet been identified.

*Saccharomyces cerevisiae* KOY.TM6P has been deposited under the Budapest treaty on the 20th of June, 2000 at the Deutsche Sammlung Mikroorganismen under deposition number DSM 13555.

*Saccharomyces cerevisiae* KOY.TM4P has been deposited under the Budapest treaty on the 6 of November, 2000 at the Deutsche Sammlung Mikroorganismen under deposition number DSM 13832.

### Growth characterisation

### Results

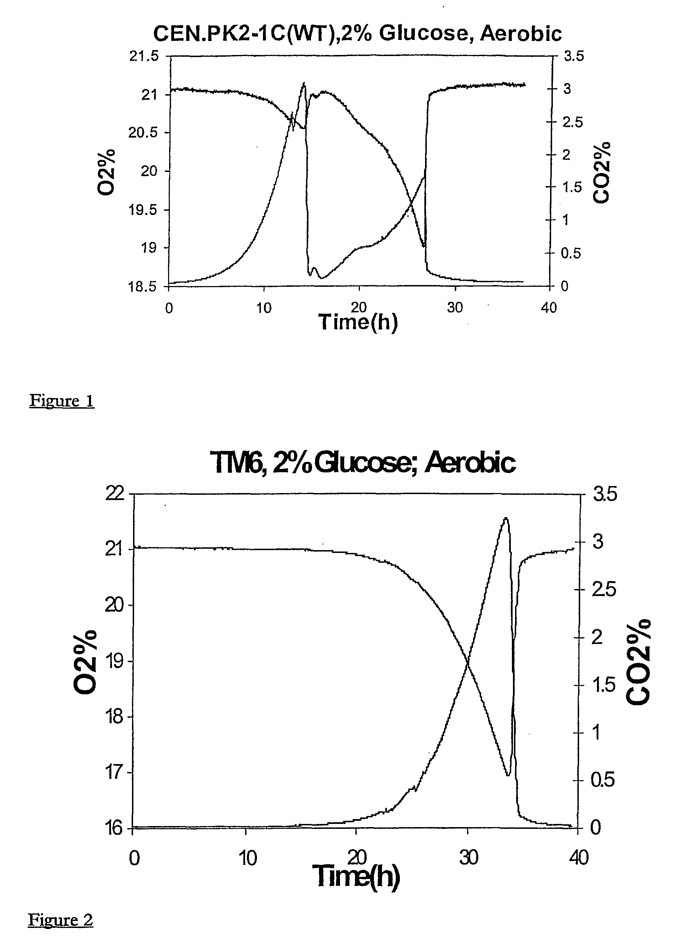

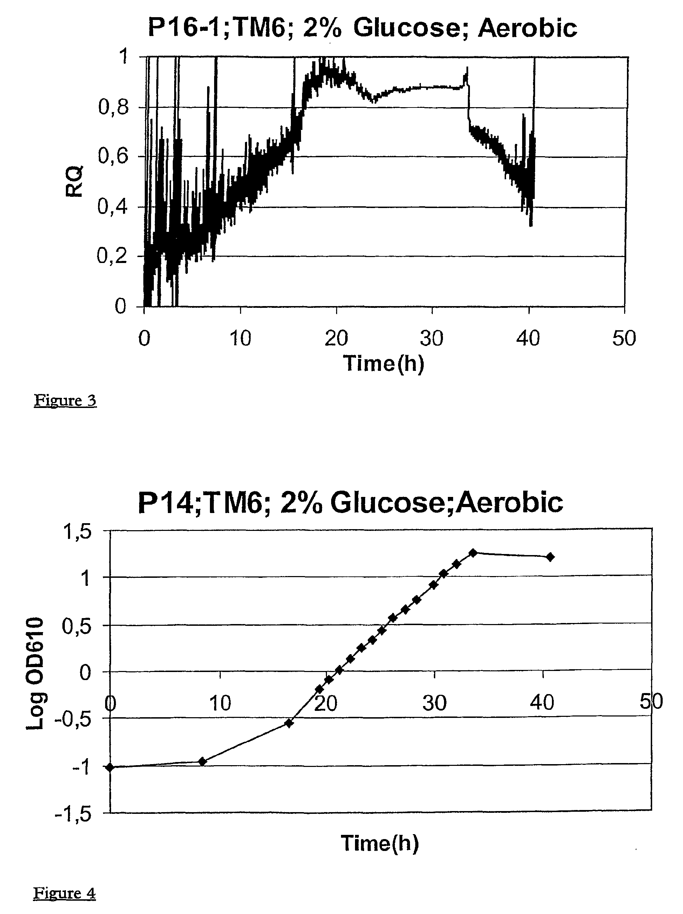

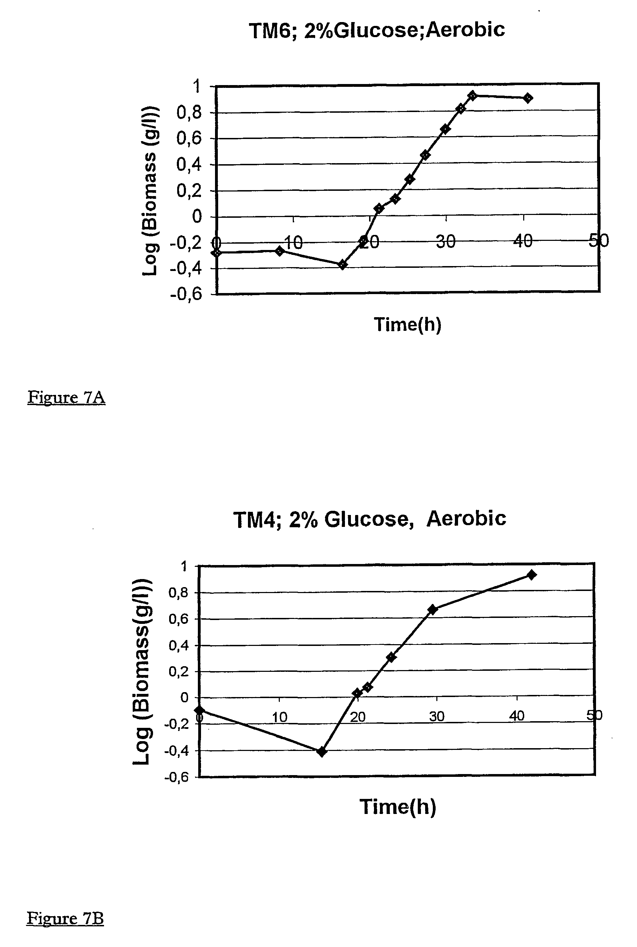

Aerobic growth: A diauxic growth behaviour typical for *S. cerevisiae* during aerobic batch cultivation on glucose is shown in Figure 1 and was performed as described previously, ie. in the medium used by Verduyn *et al.* (1992, supra) at pH 5.00 in the presence of 5mM glucose, with a starting culture of 0.3 - 0.7 g dry biomass in 1.51 fermentors, stirred at 1500rpm and an air inflow of 0.5 volume of air per vessel volume per minute (vvm). The first phase observed in Figure 1 is due to consumption of glucose with the concomitant production of ethanol and biomass. During the second phase, ethanol serves as the main carbon and energy source. However, during growth of KOY.TM6P a single growth phase is observed (Figure 2). Apparently, the sugar is immediately converted to carbon dioxide and water without any formation of ethanol, i.e. de-repressed conditions are prevalent. During these conditions a generation time of 3.8 h was obtained. This may be calculated from Figure 7 which shows the semi-log plot of biomass versus time. The maximal ethanol concentration was less than 0.12 g/l when using a glucose concentration of 20 g/l. Also other common by-products such as glycerol and acetate were found in negligible amounts. De-repressed conditions was also verified by the fact that on-line measurements of oxygen consumption rate and carbon dioxide formation rate revealed a respiratory quotient (RQ = carbon dioxide formation rate / oxygen consumption rate) close to 1. Figure 3.

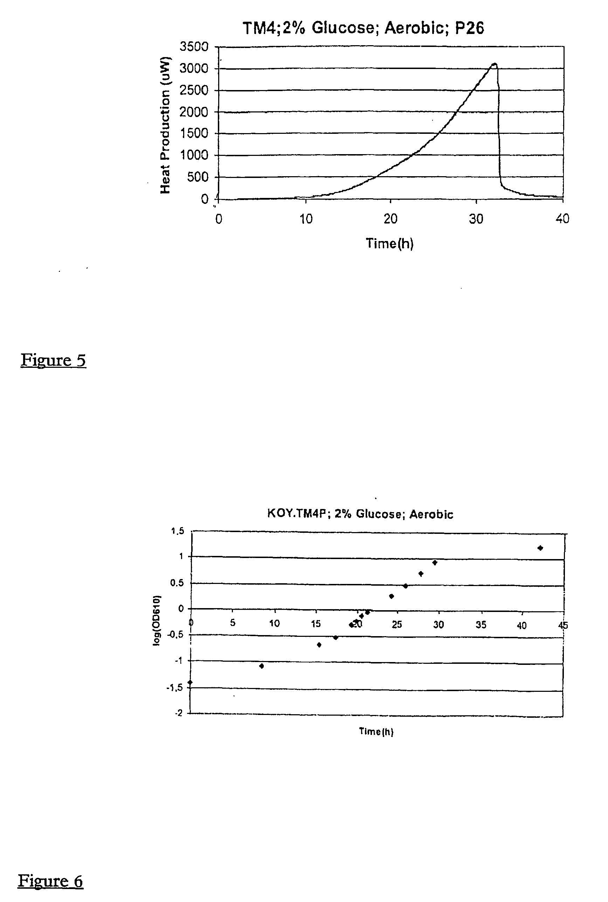

KOY.TM4P has also shown growth behaviour very similar to KOY.TM6P (Figure 5). The generation time calculated from the semi-log plot of the optical density versus time has been shown to be 3 to 3.9 h (Figure 6). The maximum ethanol production measured was 0.15 g/l when using 20 g/l of glucose in the medium. As with KOY.TM6P glycerol and acetic acid were found at very low concentrations.

Anaerobic growth: The strain, KOY.TM6P, is able to produce ethanol during anaerobic conditions. It seems, however, that after an initial phase of fairly high activity (as indicated by the specific growth and ethanol production rates) an as yet unidentified factor becomes limiting. This factor can not be such as the essential sterol ergosterol, since supplements that are known to be needed were added to the culture.

### Up-take kinetics

Glucose uptake assays has been performed using radiolabelled glucose (Walsh *et al*., 1994, supra). Cells were grown in a defined medium containing 2% glucose. Cells were harvested at OD₆₁₀=1. Cells were washed and resuspended in 100mM potassium phosphate buffer to a protein concentration of approximately 8-15mg/ml. Cells were incubated with radiolabelled glucose for about 5 sec and the amount of intracellular glucose was determined by liquid scintillation counter.

The *HXT1*-expressing strain (expressing the most abundant low affinity transport protein) showed similar uptake kinetics as the low affinity uptake component in the wild type. Surprisingly, the *HXT7*-expressing strain, which expresses the most abundant high affinity protein, showed about the same growth behaviour as the wild type and the *HXT1* -expressing strain (data not shown). The only significant difference was that the latter had a much sharper transition during the diauxic phase when the culture shifts from respiro-fermentative to respiratory growth. In other words, the much lower Vₘₐₓ of the uptake system in the *HXT7* strain compared to that of the wild type and the *HXT1* strain does not seem to restrict glucose consumption or the growth rate. It rather seems that the Kₘ for uptake displayed by the strain is the factor determining a smooth metabolic shift from glucose to another carbon and energy source. However, even lower Vₘₐₓ values, as obtained for the TM strains, seem to limit growth, resulting in diminished growth rates (data not shown).

**Table 1. Up-take kinetic data with glucose as substrate.**

| **Strain** | **Kₘ (mM)** | **Vₘₐₓ (nmol/min mg protein)** |
|---|---|---|
| WT | 67±14^{b} | 818±93^{b} |
| (CEN.PK)^{a} | 1.2±1.2^{b} | 80±80^{b} |
| HXT1 | 50-100 | 690 |
| HXT7 | 2-3 | 80 |
| TM1 | 2-3 | 35 |
| TM4 | 4-6 | 80 |
| TM6 | 7 | 38 |
| TM12 | 60 | 150 |

| | | |
|---|---|---|
| ^{a} Literature data (Diderich et al., 2000, in "Animating the cellular map", Stellenbosch University Press, South Africa, IBSN 0-7972-0776-7, p271-275) ^{b} The higher values refer to the low affinity uptake system and the lower values to the high affinity uptake system. | | |

The results obtained for the various strains described in this Example may be summarized as follows:

| | **Wild-type** | **HXT1** | **HXT7** | **TM4** | **TM6** |
|---|---|---|---|---|---|
| **Generation time (h)** | 2 | 2.5 | 2.7 | 3-3.9 | 3.8 |
| **Growth yield (biomass/g glucose)** | 0.3-0.5 | 0.3-0.35 | 0.33-0.38 | 0.34-0.37 | 0.41 |
| **Glucose consumption (mmol glucose/ (g biomass.h))** | 10 | 10 | <10 | 2.5 | 3-4 |
| **O₂ consumption rate (mmol O₂/ (g biomass.h))** | 2-2.5 | 3-4 | 2.2-6.1* | n.d. | 3-4.5 |
| **ethanol production (g/l)** | 7 | 6.2 | 5.2 | 0.15 | 0.12 |

| | | | | | |
|---|---|---|---|---|---|
| * Reflects continuous increase during the batch culture. n.d.: not determined | | | | | |

Sequence No. 1 (SEQ. ID NO. 17 in the Sequence Listing):
   Hexose transporter region of *Saccharomyces cerevisiae* KOY.TM6P bp 1-741 of HXT1 and bp 742-1713 of HXT7.
Sequence No. 2 (SEQ. ID NO. 18 in the Sequence Listing):
   Hexose transporter region of *Saccharomyces cerevisiae* KOY.TM4P bp 1-551 of HXT1 and bp 552-1713 of HXT7.
Sequence No. 3 (SEQ ID NO. 19 in the Sequence Listing): TM4 - nucleotide sequence
Sequence No. 4 (SEQ ID NO. 20 in the Sequence Listing): TM6 - nucleotide sequence
Sequence No. 5 (SEQ ID NO. 21 in the Sequence Listing): Expression cassette sequence - nucleotide sequence

## Claims

1. A modified *Saccharomyces cerevisiae* yeast which produces lower levels of ethanol than wild-type yeast under aerobic conditions and saccharide concentrations of 5mM or more and which exhibits a growth rate of at least 30% of the wild-type yeast, wherein said yeast contains a chimeric nucleotide sequence (a chimeric construct) which is stably transformed into the genetic material of the yeast, wherein the construct comprises a sequence having the form
A-B
wherein
A is a first sequence comprising nucleotide bases w to x;
B is a second sequence comprising nucleotide bases (x+y) to z;
wherein
A is from a nucleotide sequence encoding a low affinity saccharide transporter HXT-1 or HXT-3 of the yeast *Saccharomyces* or a sequence which hybridizes to the encoding sequence under non-stringent binding conditions of 6 x SSC/50% formamide at room temperature and washing under conditions of high stringency or a sequence which has at least 95% sequence identity to the encoding sequence or a sequence complementary to any of the aforesaid sequences
and
B is from a nucleotide sequence encoding a high affinity saccharide transporter HXT-2, 4, 6, 7 or GAL2P of the yeast *Saccharomyces* or a sequence which hybridizes to the encoding sequence under non-stringent binding conditions of 6 x SSC/50% formamide at room temperature and washing under conditions of high stringency or a sequence which has at least 95% sequence identity to the encoding sequence or a sequence complementary to any of the aforesaid sequences
and
the values w and x are first and second nucleotide positions within the nucleotide sequence of said low affinity saccharide transporter and (x+y) and z are first and second nucleotide positions within the nucleotide sequence of said high affinity saccharide transporter, and;
w is 1 to 50;
x is a number from 400 to 900;
y is less than 3; and
z is from 1600 to 1713,
wherein said yeast produces less than 50% ethanol compared to the wild-type yeast.

2. A yeast as claimed in claim 1 wherein said saccharide transporter is a glucose transporter.

3. A yeast as claimed in claim 1 or 2 wherein said saccharide concentration is 2% glucose.

4. A yeast as claimed in any one of claims 1 to 3 wherein said yeast produces less than 0.6 g/l ethanol.

5. A yeast as claimed in any one of claims 1 to 3 wherein said yeast produces less than 0.25 g/l ethanol.

6. A yeast as claimed in claim 5 wherein said yeast produces less than 0.12 g/l ethanol.

7. A yeast as claimed in any one of claims 1 to 6 wherein said yeast exhibits a growth rate of at least 50% of the wild-type yeast.

8. A yeast as claimed in claim 7 wherein said yeast exhibits a growth rate of at least 70% of the wild-type yeast.

9. A yeast as claimed in any one of claims 1 to 8 wherein said construct comprises sequences from HXT-1 and HXT 7 or a sequence which hybridizes to the encoding sequence under the conditions defined in claim 1 or has at least 95% sequence identity thereto or a complementary sequence thereof.

10. A yeast as claimed in any one of claims 1 to 9 wherein x is a position in the nucleotide sequence encompassed by the third to ninth transmembrane stretches of HXT1 or HXT7 or a sequence which hybridizes to the encoding sequence under the conditions defined in claim 1 or has at least 95% sequence identity thereto or a complementary sequence thereof.

11. A yeast as claimed in any one of claims 1 to 10 wherein x =525-575 and/or 720-770.

12. A yeast as claimed in any one of claims 1 to 11 wherein x = 551 or 741, w and y = 1 and z = 1713.

13. A yeast as claimed in any one of claims 1 to 12 wherein said chimeric construct encodes a chimeric polypeptide with a total of 10 to 14 transmembrane domains.

14. A yeast as claimed in any one of claims 1 to 13 wherein said construct comprises:
nucleotides 1-449 from HXT-1 and nucleotides 450-1713 from HXT-7;
nucleotides 1-551 from HXT-1 and nucleotides 552-1713 from HXT-7;
nucleotides 1-627 from HXT-1 and nucleotides 628-1713 from HXT-7;
or a nucleic acid molecule which hybridizes to said construct under the conditions defined in claim 1 or has at least 95% sequence identity to the sequence of said construct.

15. A yeast as claimed in any one of claims 1 to 14 wherein the yeast cell into which said chimeric construct is introduced is a null strain without saccharide transporting properties.

16. A yeast as claimed in any one of claims 1 to 15 wherein said chimeric construct comprises the nucleotide sequence encoding Sequence Nos. 1 or 2 or comprises the nucleotide sequence of Sequence Nos. 3 or 4 or a molecule which hybridizes to said construct or to Sequence Nos. 3 or 4 under the conditions defined in claim 1, or has at least 95% identity to the sequence of said construct or to Sequence Nos. 3 or 4.

17. A yeast as claimed in claim 15 or 16 denoted *Saccharomyces cerevisiae* KOY.TM4P or KOY.TM6P having the deposition number DSM (Deutsche Sammlung Mikroorganismen) 13832 or DSM 13555, respectively.

18. A nucleic acid molecule comprising the chimeric nucleotide sequence as defined in any one of claims 1 to 17 or a complementary sequence thereto.

19. A vector or host cell comprising the nucleic acid molecule as defined in claim 18.

20. A polypeptide comprising a sequence encoded by the nucleic acid molecule as defined in claim 18 wherein said polypeptide comprises a functional chimeric saccharide transporter.

21. A method of inserting exogenous genetic material into a yeast cell as defined in any one of claims 1 to 17 to provide a yeast cell which produces a product, said method comprising at least the steps of introducing said material into said cell, wherein said exogenous material encodes said product or portion thereof or encodes a polypeptide or part thereof which facilitates the direct or indirect production of said product or portion thereof, or which affects the expression of said polypeptide or product.

22. A method as claimed in claim 21 wherein said exogenous genetic material to be introduced into said cell is contained within a vector.

23. A method as claimed in claim 21 or 22 wherein said product is a high or low molecular metabolite, a bulk or fine chemical, a food stuff, a functional food or a therapeutic agent.

24. A method as claimed in claim 21 or 22 wherein said product is an amino acid, a peptide, a polypeptide, a sugar, a small polyol or CO₂.

25. A yeast cell as claimed in any one of claims 1 to 17 into which exogenous genetic material as defined in any one of claims 21 to 24 has been inserted to provide a yeast cell which produces a product.

26. A method of preparing a product, comprising growing yeast cells as defined in any one of claims 1 to 17 or 25 under aerobic conditions in the presence of high saccharide concentrations.

27. A method as claimed in claim 26, additionally comprising the step of isolating the product thus formed.

28. A method as claimed in claim 26 or 27 wherein said product is a low alcohol or non-alcoholic beverage.

29. A method as claimed in any one of claims 26 to 28 wherein said cells are grown in the presence of glucose.

## Patentansprüche

1. Veränderte Hefe *Saccharomyces cerevisiae,* welche unter aeroben Bedingungen im Vergleich zum Wildtyp geringere Mengen an Ethanol und Saccharidkonzentrationen von mindestens 5 mM erzeugt und welche eine Wachstumsrate von mindestens 30 % der Wildtyp-Hefe zeigt, wobei die Hefe eine chimäre Nukleotidsequenz (ein chimäres Konstrukt) enthält, welche stabil in das genetische Material der Hefe transformiert ist, wobei das Konstrukt eine Sequenz umfasst mit der Form
A-B,
wobei
A eine erste Sequenz ist, umfassend Nukleotidbasen w bis x;
B eine zweite Sequenz ist, umfassend Nukleotidbasen (x+y) bis z;
wobei
A aus einer Nukleotidsequenz stammt, codierend für einen niederaffinen Saccharidtransporter HXT-1 oder HXT-3 der Hefe *Saccharomyces* oder für eine Sequenz, welche mit der kodierenden Sequenz unter nicht stringenten Bindungsbedingungen mit 6 x SSC/50 % Formamid bei Raumtemperatur und Waschen unter hoch stringenten Bedingungen hybridisiert, oder für eine Sequenz, welche eine mindestens 95 %ige Sequenzidentität zu der codierenden Sequenz aufweist, oder eine Sequenz, die komplementär zu einer der zuvor erwähnten Sequenzen ist, ,
und
B aus einer Nukleotidsequenz stammt, codierend für einen hochaffinen Saccharidtransporter HXT-2, 4, 6, 7 oder GAL2P der Hefe *Saccharomyces* oder für eine Sequenz, welche mit der codierenden Sequenz unter nicht stringenten Bindungsbedingungen mit 6 x SSC/50 % Formamid bei Raumtemperatur und Waschen unter hoch stringenten Bedingungen hybridisiert, oder für eine Sequenz, welche eine mindestens 95 %ige Sequenzidentität mit der codierenden Sequenz aufweist, oder einer Sequenz, die komplementär zu einer der zuvor erwähnten Sequenzen ist,
und
die Werte w und x eine erste und zweite Nukleotidposition innerhalb der Nukleotidsequenz des niederaffinen Saccharidtransporters bezeichnen und (x+y) und z eine erste und zweite Nukleotidposition innerhalb der Nukleotidsequenz des hochaffinen Saccharidtransporters bezeichnen und
w 1 bis 50 ist;
x eine Zahl von 400 bis 900 ist,
y weniger als 3 ist; und
z von 1600 bis 1713 reicht,
wobei die Hefe im Vergleich zu der Wildtyp-Hefe weniger als 50 % Ethanol erzeugt.

2. Hefe nach Anspruch 1, wobei der Saccharidtransporter ein Glukosetransporter ist.

3. Hefe nach Anspruch 1 oder 2, wobei die Saccharidkonzentration 2 % Glukose beträgt.

4. Hefe nach einem der Ansprüche 1 bis 3, wobei die Hefe weniger als 0,6 g/l Ethanol erzeugt.

5. Hefe nach einem der Ansprüche 1 bis 3, wobei die Hefe weniger als 0,25 g/l Ethanol erzeugt.

6. Hefe nach Anspruch 5, wobei die Hefe weniger als 0,12 g/l Ethanol erzeugt.

7. Hefe nach einem der Ansprüche 1 bis 6, wobei die Hefe eine Wachstumsrate von mindestens 50 % der Wildtyp-Hefe aufweist.

8. Hefe nach Anspruch 7, wobei die Hefe eine Wachstumsrate von mindestens 70 % der Wildtyp-Hefe aufweist.

9. Hefe nach einem der Ansprüche 1 bis 8, wobei das Konstrukt Sequenzen von HXT-1 und HXT-7 oder eine Sequenz, welche mit der codierenden Sequenz unter den in Anspruch 1 definierten Bedingungen hybridisiert oder eine mindestens 95 %ige Sequenzidentität mit dieser aufweist, oder einer Komplementärsequenz davon umfasst.

10. Hefe nach einem der Ansprüche 1 bis 9, wobei x eine Position in der Nukleotidsequenz ist, welche in dem dritten bis neunten Transmembranabschnitt von HXT-1 oder HXT-7 enthalten ist, oder in einer Sequenz, welche mit der kodierenden Sequenz unter den in Anspruch 1 definierten Bedingungen hybridisiert oder eine mindestens 95 %ige Sequenzidentität mit dieser aufweist, oder in einer Komplementärsequenz davon.

11. Hefe nach einem der Ansprüche 1 bis 10, wobei x = 525-575 und/oder 720-770.

12. Hefe nach einem der Ansprüche 1 bis 11, wobei x = 551 oder 741, w und y = 1 und z = 1713.

13. Hefe nach einem der Ansprüche 1 bis 12, wobei das chimäre Konstrukt für ein chimäres Polypeptid mit insgesamt 10 bis 14 Transmembrandomänen codiert.

14. Hefe nach einem der Ansprüche 1 bis 13, wobei das Konstrukt umfasst:
Nukleotide 1-449 aus HXT-1 und Nukleotide 450-1713 aus HXT-7;
Nukleotide 1-551 aus HXT-1 und Nukleotide 552-1713 aus HXT-7;
Nukleotide 1-627 aus HXT-1 und Nukleotide 628-1713 aus HXT-7;
oder ein Nukleinsäuremolekül, welches mit dem Konstrukt unter den in Anspruch 1 definierten Bedingungen hybridisiert oder eine mindestens 95 %ige Sequenzidentität mit der Sequenz des Konstrukts aufweist.

15. Hefe nach einem der Ansprüche 1 bis 14, wobei die Hefezelle, in welche das chimäre Konstrukt eingeführt wird, ein Nullstamm ohne Saccharidtransporteigenschaften ist.

16. Hefe nach einem der Ansprüche 1 bis 15, wobei das chimäre Konstrukt die Nukleotidsequenz umfasst, die für die Sequenz Nr. 1 oder 2 codiert, oder die Nukleotidsequenz von Sequenz Nr. 3 oder 4 umfasst oder ein Molekül, welches mit dem Konstrukt oder mit Sequenz Nr. 3 oder 4 unter den in Anspruch 1 definierten Bedingungen hybridisiert oder eine mindestens 95 %ige Identität zu der Sequenz des Konstrukts oder zu Sequenz Nr. 3 oder 4 aufweist.

17. Hefe nach Anspruch 15 oder 16, welche als *Saccharomyces cerevisiae* KOY.TM4P oder KOY.TM6P bezeichnet wird, mit der Depotnummer DSM (Deutsche Sammlung von Mikroorganismen) 13832 beziehungsweise DSM 13555.

18. Nukleinsäuremolekül, umfassend die chimäre Nukleotidsequenz, wie in einem der Ansprüche 1 bis 17 definiert, oder eine Komplementärsequenz dazu.

19. Vektor oder Wirtszelle, umfassend das Nukleinsäuremolekül, wie in Anspruch 18 definiert.

20. Polypeptid, umfassend eine Sequenz, die durch das Nukleinsäuremolekül, wie in Anspruch 18 definiert, codiert wird, wobei das Polypeptid einen funktionellen chimären Saccharidtransporter umfasst.

21. Verfahren zur Insertion exogenen genetischen Materials in eine Hefezelle, wie in einem der Ansprüche 1 bis 17 definiert, um eine Hefezelle bereitzustellen, welche ein Produkt erzeugt, wobei das Verfahren mindestens die Schritte zum Einführen des Materials in die Zelle umfasst, wobei das exogene Material für das Produkt oder einen Anteil davon codiert oder für ein Polypeptid oder ein Teil davon codiert, welches die direkte oder indirekte Erzeugung des Produkts oder eines Anteils davon erleichtert, oder welches die Expression des Polypeptids oder Produkts betrifft.

22. Verfahren nach Anspruch 21, wobei das exogene genetische Material, das in die Zelle eingeführt wird, in einem Vektor enthalten ist.

23. Verfahren nach Anspruch 21 oder 22, wobei das Produkt ein hoch- oder niedermolekularer Metabolit, eine Massen- oder Feinchemikalie, ein Nahrungsmittel, ein funktionelles Nahrungsmittel oder ein therapeutisches Mittel ist.

24. Verfahren nach Anspruch 21 oder 22, wobei das Produkt eine Aminosäure, ein Peptid, ein Polypeptid, ein Zucker, ein kleines Polyol oder CO₂ ist.

25. Hefezelle nach einem der Ansprüche 1 bis 17, in welche exogenes genetisches Material, wie in einem der Ansprüche 21 bis 24 definiert, eingeführt wurde, um eine Hefezelle bereitzustellen, welche ein Produkt erzeugt.

26. Verfahren zur Erzeugung eines Produkts, umfassend Anzucht von Hefezellen, wie in einem der Ansprüche 1 bis 17 oder 25 definiert, unter aeroben Bedingungen in Gegenwart von hohen Saccharidkonzentrationen.

27. Verfahren nach Anspruch 26, zusätzlich umfassend den Schritt zur Isolierung des so gebildeten Produkts.

28. Verfahren nach Anspruch 26 oder 27, wobei das Produkt ein Getränk mit niedrigem Alkoholgehalt oder ein alkoholfreies Getränk ist.

29. Verfahren nach einem der Ansprüche 26 bis 28, wobei die Zellen in der Gegenwart von Glukose angezogen werden.

## Revendications

1. Levure *Saccharomyces cerevisiae* modifiée qui produit des taux d'éthanol inférieurs à ceux de la levure de type sauvage sous des conditions aérobies et des concentrations en saccharide de 5 mM ou plus et qui présente un taux de croissance d'au moins 30 % de la levure de type sauvage, dans laquelle ladite levure contient une séquence nucléotidique chimérique (une construction chimérique) qui est transformée de façon stable en matériel génétique de la levure, dans laquelle la construction comprend une séquence de forme
A - B
dans laquelle
A est une première séquence comprenant les bases nucléotidiques w à x ;
B est une seconde séquence comprenant les bases nucléotidiques (x+y) à z ;
dans lesquelles
A provient d'une séquence nucléotidique codant pour un transporteur de saccharide de faible affinité HXT-1 ou HXT-3 de la levure *Saccharomyces* ou une séquence qui s'hybride à la séquence codante sous des conditions de liaison astringentes de 6 x SSC / 50 % de formamide à température ambiante et de lavage sous des conditions hautement stringentes ou une séquence qui a une identité de séquences d'au moins 95 % avec la séquence codante ou une séquence complémentaire à l'une quelconque des séquences précédemment mentionnées
et
B provient d'une séquence nucléotidique codant pour un transporteur de saccharide d'affinité élevée HXT-2, 4, 6, 7 ou GAL2P de la levure *Saccharomyces* ou une séquence qui s'hybride à la séquence codante sous des conditions de liaison astringentes de 6 × SSC / 50 % de formamide à température ambiante et de lavage sous des conditions hautement stringentes ou une séquence qui a une identité de séquences d'au moins 95 % avec la séquence codante ou une séquence complémentaire à l'une quelconque des séquences précédemment mentionnées
et
les valeurs w et x sont les première et deuxième positions des nucléotides à l'intérieur de la séquence nucléotidiquedudit transporteur de saccharide de faible affinité et (x+y) et z sont les première et deuxième positions des nucléotides à l'intérieur de la séquence nucléotidiquedudit transporteur de saccharide d'affinité élevée, et ; w vaut 1 à 50 ;
x est un nombre de 400 à 900 ;
y est inférieur à 3 ; et
z vaut 1 600 à 1 713,
dans laquelle ladite levure produit moins de 50 % d'éthanol par rapport à la levure de type sauvage.

2. Levure selon la revendication 1, dans laquelle ledit transporteur de saccharide est un transporteur de glucose.

3. Levure selon la revendication 1 ou 2, dans laquelle ladite concentration en saccharide est de 2 % de glucose.

4. Levure selon l'une quelconque des revendications 1 à 3, dans laquelle ladite levure produit moins de 0,6 g/l d'éthanol.

5. Levure selon l'une quelconque des revendications 1 à 3, dans laquelle ladite levure produit moins de 0,25 g/l d'éthanol.

6. Levure selon la revendication 5, dans laquelle ladite levure produit moins de 0,12 g/l d'éthanol.

7. Levure selon l'une quelconque des revendications 1 à 6, dans laquelle ladite levure présente un taux de croissance d'au moins 50 % de la levure de type sauvage.

8. Levure selon la revendication 7, dans laquelle ladite levure présente un taux de croissance d'au moins 70 % de la levure de type sauvage.

9. Levure selon l'une quelconque des revendications 1 à 8, dans laquelle ladite construction comprend des séquences provenant de HXT-1 et HXT-7 ou une séquence qui s'hybride à la séquence codante sous des conditions définies dans la revendication 1 ou qui a une identité de séquences d'au moins 95 % avec celle-ci ou une séquence complémentaire de celle-ci.

10. Levure selon l'une quelconque des revendications 1 à 9, dans laquelle x est une position dans la séquence nuléotidique englobée par les troisième à neuvième segments transmembranaires de HXT1 ou HXT7 ou une séquence qui s'hybride à la séquence codante sous des conditions définies dans la revendication 1 ou qui a une identité de séquences d'au moins 95 % avec celle-ci ou une séquence complémentaire de celle-ci.

11. Levure selon l'une quelconque des revendications 1 à 10, dans laquelle x = 525 à 575 et/ou 720 à 770.

12. Levure selon l'une quelconque des revendications 1 à 11, dans laquelle x = 551 ou 741, w et y = 1 et z = 1 713.

13. Levure selon l'une quelconque des revendications 1 à 12, dans laquelle ladite construction chimérique code pour un polypeptide chimérique avec un total de 10 à 14 domaines transmembranaires.

14. Levure selon l'une quelconque des revendications 1 à 13, dans laquelle ladite construction comprend :
les nucléotides 1 à 449 de HXT-1 et les nucléotides 450 à 1 713 de HXT-7 ;
les nucléotides 1 à 551 de HXT-1 et les nucléotides 552 à 1 713 de HXT-7 ;
les nucléotides 1 à 627 de HXT-1 et les nucléotides 628 à 1 713 de HXT-7 ;
ou une molécule d'acide nucléique qui s'hybride à ladite construction sous les conditions définies dans la revendication 1 ou qui a une identité de séquences d'au moins 95 % avec la séquence de ladite construction.

15. Levure selon l'une quelconque des revendications 1 à 14, dans laquelle la cellule de levure dans laquelle ledit constituant chimérique est introduit est une souche vide sans propriétés de transport de saccharide.

16. Levure selon l'une quelconque des revendications 1 à 15, dans laquelle ladite construction chimérique comprend la séquence nucléotidique codant pour la séquence n° 1 ou 2 ou comprend la séquence nucléotidique de séquence n° 3 ou 4 ou une molécule qui s'hybride à ladite construction ou à la séquence n° 3 ou 4 sous les conditions définies dans la revendication 1, ou a une identité d'au moins 95 % avec la séquence de ladite construction ou avec la séquence n° 3 ou 4.

17. Levure selon la revendication 15 ou 16, appelée *Saccharomyces cerevisiae* KOY.TM4P ou KOY.TM6P, ayant respectivement le numéro de dépôt DSM (Deutsche Sammlung Mikroorganismen) 13832 ou DSM 13555.

18. Molécule d'acide nucléique comprenant la séquence nucléotidique chimérique selon l'une quelconque des revendications 1 à 17 ou une séquence complémentaire de celle-ci.

19. Vecteur ou cellule hôte comprenant la molécule d'acide nucléique selon la revendication 18.

20. Polypeptide comprenant une séquence codée par la molécule d'acide nucléique telle que définie dans la revendication 18, dans lequel ledit polypeptide comprend un transporteur de saccharide chimérique fonctionnel.

21. Procédé d'insertion de matériel génétique exogène dans une cellule de levure selon l'une quelconque des revendications 1 à 17 pour fournir une cellule de levure qui donne un produit, ledit procédé comprenant au moins les étapes d'introduction dudit matériel dans ladite cellule, dans lequel ledit matériel exogène code pour ledit produit ou une partie de celui-ci ou code pour un polypeptide ou une partie de celui-ci qui facilite la production directe ou indirecte dudit produit ou de ladite partie de celui-ci, ou qui affecte l'expression dudit polypeptide ou produit.

22. Procédé selon la revendication 21, dans lequel ledit matériel génétique exogène à introduire dans ladite cellule est contenu à l'intérieur d'un vecteur.

23. Procédé selon la revendication 21 ou 22, dans lequel ledit produit est un métabolite moléculaire élevé ou faible, un produit chimique grossier ou fin, une substance alimentaire, un aliment fonctionnel ou un agent thérapeutique.

24. Procédé selon la revendication 21 ou 22, dans lequel ledit produit est un acide aminé, un peptide, un polypeptide, un sucre, un petit polyol ou du CO₂.

25. Cellule de levure selon l'une quelconque des revendications 1 à 17, dans laquelle le matériel génétique exogène selon l'une quelconque des revendications 21 à 24 a été inséré pour fournir une cellule de levure qui donne un produit.

26. Procédé de préparation d'un produit, comprenant la croissance de cellules de levure selon l'une quelconque des revendications 1 à 17 ou 25 sous des conditions aérobies en présence de concentrations en saccharide élevées.

27. Procédé selon la revendication 26, comprenant en outre l'étape d'isolement du produit ainsi formé.

28. Procédé selon la revendication 26 ou 27, dans lequel ledit produit est un alcool inférieur ou une boisson sans alcool.

29. Procédé selon l'une quelconque des revendications 26 à 28, dans lequel lesdites cellules sont cultivées en présence de glucose.


### SEQUENCE LISTING

<110> Gothia Yeast Solutions AB
<120> Modified Yeast
<130> 42.75657
<160> 21
<170> PatentIn version 3.0
<210> 1
   <211> 72
   <212> DNA
   <213> Artificial/Unknown
<220>
   <221> misc_feature
   <222> () .. ()
   <223> Forward Primer
<400> 1
   caaagaataa acacaaaaac aaaaagtttt tttaatttta atcaaaaaat gaattcaact
   60
   cccgatctaa ta
   72
<210> 2
   <211> 36
   <212> DNA
   <213> Artificial/Unknown
<220>
   <221> misc_feature
   <222> () .. ()
   <223> Primer
<400> 2
   ggagataaaa cggcaatacc accgacacct aaacca
   36
<210> 3
   <211> 36
   <212> DNA
   <213> Artificial/Unknown
<220>
   <221> misc_feature
   <222> () .. ()
   <223> Primer
<400> 3
   tggtttaggt gtcggtggta ttgccgtttt atctcc
   36
<210> 4
   <211> 24
   <212> DNA
   <213> Artificial/Unknown
<220>
   <221> misc feature
   <222> () .. ()
   <223> Primer
<400> 4
   tttgtagacg tgggtctgca ggca
   24
<210> 5
   <211> 72
   <212> DNA
   <213> Artificial/Unknown
<220>
   <221> misc_feature
   <222> () .. ()
   <223> Primer
<400> 5
   caaagaataa acacaaaaac aaaaagtttt tttaatttta atcaaaaaat gaattcaact
   60
   cccgatctaa ta
   72
<210> 6
   <211> 41
   <212> DNA
   <213> Artificial/Unknown
<220>
   <221> misc_feature
   <222> () .. ()
   <223> Primer
<400> 6
   ctggaacaaa tgtcatacca ccaatcataa ataaggccca g
   41
<210> 7
   <211> 41
   <212> DNA
   <213> Artificial/Unknown
<220>
   <221> misc_feature
   <222> () .. ()
   <223> Primer
<400> 7
   ctgggcctta tttatgattg gtggtatgac atttgttcca g
   41
<210> 8
   <211> 24
   <212> DNA
   <213> Artificial/Unknown
<220>
   <221> misc_feature
   <222> () .. ()
   <223> Primer
<400> 8
   tttgtagacg tgggtctgca ggca
   24
<210> 9
   <211> 34
   <212> DNA
   <213> Artificial/Unknown
<220>
   <221> misc_feature
   <222> () .. ()
   <223> Primer
<400> 9
   ggactagtga tatctctcgt aggaacaatt tcgg
   34
<210> 10
   <211> 28
   <212> DNA
   <213> Artificial/Unknown
<220>
   <221> misc_feature
   <222> () .. ()
   <223> Primer
<400> 10
   ggactagttg ctctgcaata gcagcgtc
   28
<210> 11
   <211> 70
   <212> DNA
   <213> Artificial/Unknown
<220>
   <221> misc_feature
   <222> () .. ()
   <223> Primer
<400> 11
   cctattcgtc atcgcagaca gccttcatct tctcgagata acacctggag cgcgcgtttc 60
   ggtgatgacg 70
<210> 12
   <211> 73
   <212> DNA
   <213> Artificial/Unknown
<220>
   <221> misc_feature
   <222> () .. ()
   <223> Primer
<400> 12
   aagtttcttt gtctccgtcc cactcaactt tctgagaaca aatgatccat tttttgatta 60
   aaattaaaaa aac 73
<210> 13
   <211> 22
   <212> DNA
   <213> Artificial/Unknown
<220>
   <221> misc_feature
   <222> () .. ()
   <223> Primer
<400> 13
   cctgcgtgtt cttctgaggt tc 22
<210> 14
   <211> 24
   <212> DNA
   <213> Artificial/Unknown
<220>
   <221> misc_feature
   <222> () .. ()
   <223> Primer
<400> 14
   tttgtagacg tgggtctgca ggca
   24
<210> 15
   <211> 74
   <212> DNA
   <213> Artificial/Unknown
<220>
   <221> misc_feature
   <222> () .. ()
   <223> Primer
<400> 15
   caaagaataa acacaaaaac aaaaagtttt tttaatttta atcaaaaaat gtccacaaaa
   60
   tcatatacca gtag
   74
<210> 16
   <211> 72
   <212> DNA
   <213> Artificial/Unknown
<220>
   <221> misc_feature
   <222> () .. ()
   <223> Primer
<400> 16
   gcacaaatta gagcgtgatc atgaattaat aaaagtgttc gcaaattaat ggggagcgct
   60
   gattctcttt tg
   72
<210> 17
   <211> 570
   <212> PRT
   <213> Artificial/Unknown
<220>
   <221> misc_feature
   <222> () .. ()
   <223> Chimera of HXT1 (1 to 741) and HXT7 (742 to 1713). Tyr at 2
   79 can
<400> 17
   Met Asn Ser Thr Pro Asp Leu Ile Ser Pro Gln Lys Ser Asn Ser Ser
   1               5                   10                  15
   Asn Ser Tyr Glu Leu Glu Ser Gly Arg Ser Lys Ala Met Asn Thr Pro
               20                  25                  30
   Glu Gly Lys Asn Glu Ser Phe His Asp Asn Leu Ser Glu Ser Gln Val
           35                  40                  45
   Gln Pro Ala Val Ala Pro Pro Asn Thr Gly Lys Gly Val Tyr Val Thr
       50                  55                  60
   Val Ser Ile Cys Cys Val Met Val Ala Phe Gly Gly Phe Ile Phe Gly
   65                  70                  75                  80
   Trp Asp Thr Gly Thr Ile Ser Gly Phe Val Ala Gln Thr Asp Phe Leu
                   85                  90                  95
   Arg Arg Phe Gly Met Lys His His Asp Gly Ser His Tyr Leu Ser Lys
   100 105 110
   Val Arg Thr Gly Leu Ile Val Ser Ile Phe Asn Ile Gly Cys Ala Ile
   115 120 125
   Gly Gly Ile Val Leu Ala Lys Leu Gly Asp Met Tyr Gly Arg Arg Ile
   130 135 140
   Gly Leu Ile Val Val Val Val Ile Tyr Thr Ile Gly Ile Ile Ile Gln
   145 150 155 160
   Ile Ala Ser Ile Asn Lys Trp Tyr Gln Tyr Phe Ile Gly Arg Ile Ile
   165 170 175
   Ser Gly Leu Gly Val Gly Gly Ile Thr Val Leu Ser Pro Met Leu Ile
   180 185 190
   Ser Glu Val Ala Pro Ser Glu Met Arg Gly Thr Leu Val Ser Cys Tyr
   195 200 205
   Gln Val Met Ile Thr Leu Gly Ile Phe Leu Gly Tyr Cys Thr Asn Phe
   210 215 220
   Gly Thr Lys Asn Tyr Ser Asn Ser Val Gln Trp Arg Val Pro Leu Gly
   225 230 235 240
   Leu Cys Phe Ala Trp Ala Leu Phe Met Ile Gly Gly Met Thr Phe Val
   245 250 255
   Pro Glu Ser Pro Arg Tyr Leu Ala Glu Val Gly Lys Ile Glu Glu Ala
   260 265 270
   Lys Arg Ser Ile Ala Val Tyr Asn Lys Val Ala Val Asp Asp Pro Ser
   275 280 285
   Val Leu Ala Glu Val Glu Ala Val Leu Ala Gly Val Glu Ala Glu Lys
   290 295 300
   Leu Ala Gly Asn Ala Ser Trp Gly Glu Leu Phe Ser Ser Lys Thr Lys
   305 310 315 320
   Val Leu Gln Arg Leu Ile Met Gly Ala Met Ile Gln Ser Leu Gln Gln
   325 330 335
   Leu Thr Gly Asp Asn Tyr Phe Phe Tyr Tyr Gly Thr Thr Ile Phe Lys
   340 345 350
   Ala Val Gly Leu Ser Asp Ser Phe Glu Thr Ser Ile Val Leu Gly Ile
   355 360 365
   Val Asn Phe Ala Ser Thr Phe Val Gly Ile Tyr Val Val Glu Arg Tyr
       370                 375                 380
   Gly Arg Arg Thr Cys Leu Leu Trp Gly Ala Ala Ser Met Thr Ala Cys
   385                 390                 395                 400
   Met Val Val Tyr Ala Ser Val Gly Val Thr Arg Leu Trp Pro Asn Gly
                   405                 410                 415
   Gln Asp Gln Pro Ser Ser Lys Gly Ala Gly Asn Cys Met Ile Val Phe
               420                 425                 430
   Ala Cys Phe Tyr Ile Phe Cys Phe Ala Thr Thr Trp Ala Pro Ile Pro
           435                 440                 445
   Tyr Val Val Val Ser Glu Thr Phe Pro Leu Arg Val Lys Ser Lys Ala
       450                 455                 460
   Met Ser Ile Ala Thr Ala Ala Asn Trp Leu Trp Gly Phe Leu Ile Gly
   465                 470                 475                 480
   Phe Phe Thr Pro Phe Ile Thr Gly Ala Ile Asn Phe Tyr Tyr Gly Tyr
                   485                 490                 495
   Val Phe Met Gly Cys Leu Val Phe Met Phe Phe Tyr Val Leu Leu Val
               500                 505                 510
   Val Pro Glu Thr Lys Gly Leu Thr Leu Glu Glu Val Asn Thr Met Trp
           515                 520                 525
   Glu Glu Gly Val Leu Pro Trp Lys Ser Ala Ser Trp Val Pro Pro Ser
       530                 535                 540
   Arg Arg Gly Ala Asn Tyr Asp Ala Glu Glu Met Thr His Asp Asp Lys
   545                 550                 555                 560
   Pro Leu Tyr Lys Arg Met Phe Ser Thr Lys
                   565                 570
<210> 18
   <211> 570
   <212> PRT
   <213> Artificial/Unknown
<220>
   <221> misc_feature
   <222> ()..()
   <223> Chimera of HXT1 (1 to 551) and HXT7 (552 to 1713)
<400> 18
   Met Asn Ser Thr Pro Asp Leu Ile Ser Pro Gln Lys Ser Asn Ser Ser
   1               5                   10                  15
   Asn Ser Tyr Glu Leu Glu Ser Gly Arg Ser Lys Ala Met Asn Thr Pro
               20                  25                  30
   Glu Gly Lys Asn Glu Ser Phe His Asp Asn Leu Ser Glu Ser Gln Val
           35                  40                  45
   Gln Pro Ala Val Ala Pro Pro Asn Thr Gly Lys Gly Val Tyr Val Thr
       50                  55                  60
   Val Ser Ile Cys Cys Val Met Val Ala Phe Gly Gly Phe Ile Phe Gly
   65                  70                  75                  80
   Trp Asp Thr Gly Thr Ile Ser Gly Phe Val Ala Gln Thr Asp Phe Leu
                   85                  90                  95
   Arg Arg Phe Gly Met Lys His His Asp Gly Ser His Tyr Leu Ser Lys
               100                 105                 110
   Val Arg Thr Gly Leu Ile Val Ser Ile Phe Asn Ile Gly Cys Ala Ile
           115                 120                 125
   Gly Gly Ile Val Leu Ala Lys Leu Gly Asp Met Tyr Gly Arg Arg Ile
       130                 135                 140
   Gly Leu Ile Val Val Val Val Ile Tyr Thr Ile Gly Ile Ile Ile Gln
   145                 150                 155                 160
   Ile Ala Ser Ile Asn Lys Trp Tyr Gln Tyr Phe Ile Gly Arg Ile Ile
                   165                 170                 175
   Ser Gly Leu Gly Val Gly Gly Ile Ala Val Leu Ser Pro Met Leu Ile
               180                 185                 190
   Ser Glu Val Ser Pro Lys His Leu Arg Gly Thr Leu Val Ser Cys Tyr
           195                 200                 205
   Gln Leu Met Ile Thr Ala Gly Ile Phe Leu Gly Tyr Cys Thr Asn Phe
       210                 215                 220
   Gly Thr Lys Asn Tyr Ser Asn Ser Val Gln Trp Arg Val Pro Leu Gly
   225                 230                 235                 240
   Leu Cys Phe Ala Trp Ala Leu Phe Met Ile Gly Gly Met Thr Phe Val
                   245                 250                 255
   Pro Glu Ser Pro Arg Tyr Leu Ala Glu Val Gly Lys Ile Glu Glu Ala
               260                 265                 270
   Lys Arg Ser Ile Ala Val Ser Asn Lys Val Ala Val Asp Asp Pro Ser
           275                 280                 285
   Val Leu Ala Glu Val Glu Ala Val Leu Ala Gly Val Glu Ala Glu Lys
   290 295 300
   Leu Ala Gly Asn Ala Ser Trp Gly Glu Leu Phe Ser Ser Lys Thr Lys
   305 310 315 320
   Val Leu Gln Arg Leu Ile Met Gly Ala Met Ile Gln Ser Leu Gln Gln
   325 330 335
   Leu Thr Gly Asp Asn Tyr Phe Phe Tyr Tyr Gly Thr Thr Ile Phe Lys
   340 345 350
   Ala Val Gly Leu Ser Asp Ser Phe Glu Thr Ser Ile Val Leu Gly Ile
   355 360 365
   Val Asn Phe Ala Ser Thr Phe Val Gly Ile Tyr Val Val Glu Arg Tyr
   370 375 380
   Gly Arg Arg Thr Cys Leu Leu Trp Gly Ala Ala Ser Met Thr Ala Cys
   385 390 395 400
   Met Val Val Tyr Ala Ser Val Gly Val Thr Arg Leu Trp Pro Asn Gly
   405 410 415
   Gln Asp Gln Pro Ser Ser Lys Gly Ala Gly Asn Cys Met Ile Val Phe
   420 425 430
   Ala Cys Phe Tyr Ile Phe Cys Phe Ala Thr Thr Trp Ala Pro Ile Pro
   435 440 445
   Tyr Val Val Val Ser Glu Thr Phe Pro Leu Arg Val Lys Ser Lys Ala
   450 455 460
   Met Ser Ile Ala Thr Ala Ala Asn Trp Leu Trp Gly Phe Leu Ile Gly
   465 470 475 480
   Phe Phe Thr Pro Phe Ile Thr Gly Ala Ile Asn Phe Tyr Tyr Gly Tyr
   485 490 495
   Val Phe Met Gly Cys Leu Val Phe Met Phe Phe Tyr Val Leu Leu Val
   500 505 510
   Val Pro Glu Thr Lys Gly Leu Thr Leu Glu Glu Val Asn Thr Met Trp
   515 520 525
   Glu Glu Gly Val Leu Pro Trp Lys Ser Ala Ser Trp Val Pro Pro Ser
   530 535 540
   Arg Arg Gly Ala Asn Tyr Asp Ala Glu Glu Met Thr His Asp Asp Lys
   545 550 555 560
   Pro Leu Tyr Lys Arg Met Phe Ser Thr Lys
   565 570
<210> 19
   <211> 1713
   <212> DNA
   <213> Artificial/Unknown
<220>
   <221> misc_feature
   <222> () .. ()
   <223> Chimera of HXT1 (1 to 551) and HXT7 (552 to 1713)
<400> 19
   atgaattcaa ctcccgatct aatatctcct cagaaatcca attcatccaa ctcatatgaa 60
   ttggaatctg gtcgttcaaa ggccatgaat actccagaag gtaaaaatga aagttttcac 120
   gacaacttaa gtgaaagtca agtgcaaccc gccgttgccc ctccaaacac cggaaaaggt 180
   gtctacgtaa cggtttctat ctgttgtgtt atggttgctt tcggtggttt catatttgga 240
   tgggatactg gtaccatttc tggttttgtt gctcaaactg attttctaag aagatttggt 300
   atgaagcacc acgacggtag tcattacttg tccaaggtga gaactggttt aattgtctct 360
   atttttaaca ttggttgtgc cattggtggt atcgtcttag ccaagctagg tgatatgtat 420
   ggtcgtagaa tcggtttgat tgtcgttgta gtaatctaca ctatcggtat cattattcaa 480
   atagcctcga tcaacaagtg gtaccaatat ttcattggta gaattatctc tggtttaggt 540
   gtcggtggta ttgccgtttt atctcctatg ttgatttctg aagtatcccc aaagcattta 600
   aggggtactt tagtctcttg ctaccaattg atgattactg ccggtatttt cttgggttac 660
   tgtaccaact tcggtactaa gaactactcc aactctgtgc aatggagagt tccattaggt 720
   ttgtgttttg cctgggcttt gtttatgatt ggtggtatga catttgttcc agagtctcca 780
   cgttatttgg ctgaagtcgg taagatcgaa gaagccaaac gttctattgc cgtttctaac 840
   aaggttgctg ttgatgatcc atctgttttg gctgaagtcg aagctgtctt ggctggtgta
   900
   gaggcagaga aattagctgg taatgcatcc tggggtgaat tgtttagtag caagacaaag
   960
   gtccttcagc gtttgatcat gggtgctatg attcaatctc tacaacaatt gacaggtgat
   1020
   aactatttct tctactatgg tactactatt ttcaaggctg ttggtttgag tgactctttc
   1080
   gaaacctcta ttgtcttggg tattgttaac tttgcttcca cctttgttgg tatttacgtt
   1140
   gttgagagat atggtcgtcg tacttgtttg ctatggggtg ctgcatccat gactgcttgt
   1200
   atggttgtct atgcttccgt gggtgtcacc agattatggc caaatggtca agaccaacca
   1260
   tcttccaagg gtgctggtaa ctgtatgatt gtctttgcct gtttctatat tttctgtttt
   1320
   gctactacat gggctccaat tccttatgtc gttgtttctg aaactttccc attgagagtc
   1380
   aagtctaagg ctatgtctat tgctacagct gctaattggt tgtggggttt cttgattggt
   1440
   ttcttcactc catttattac tggtgctatt aacttctact acggttacgt tttcatgggc
   1500
   tgtttggtct tcatgttctt ctatgttttg ttagttgttc cagaaactaa gggtttgact
   1560
   ttggaagaag tcaacaccat gtgggaagaa ggtgttctac catggaagtc tgcctcatgg
   1620
   gttccaccat ccagaagagg tgccaactac gacgctgaag aaatgactca cgatgacaag
   1680
   ccattgtaca agagaatgtt cagcaccaaa taa
   1713
<210> 20
   <211> 1713
   <212> DNA
   <213> Artificial/Unknown
<220>
   <221> misc_feature
   <222> () .. ()
   <223> Chimera of HXT1 (1 to 741) and HXT7 (742 to 1713)
<400> 20
   atgaattcaa ctcccgatct aatatctcct cagaaatcca attcatccaa ctcatatgaa 60
   ttggaatctg gtcgttcaaa ggccatgaat actccagaag gtaaaaatga aagttttcac 120
   gacaacttaa gtgaaagtca agtgcaaccc gccgttgccc ctccaaacac cggaaaaggt 180
   gtctacgtaa cggtttctat ctgttgtgtt atggttgctt tcggtggttt catatttgga 240
   tgggatactg gtaccatttc tggttttgtt gctcaaactg attttctaag aagatttggt 300
   atgaagcacc acgacggtag tcattacttg tccaaggtga gaactggttt aattgtctct 360
   atttttaaca ttggttgtgc cattggtggt atcgtcttag ccaagctagg tgatatgtat 420
   ggtcgtagaa tcggtttgat tgtcgttgta gtaatctaca ctatcggtat cattattcaa 480
   atagcctcga tcaacaagtg gtaccaatat ttcattggta gaattatctc tggtttaggt 540
   gtcggtggta tcacagtttt atctcccatg ctaatatctg aggtcgcccc cagtgaaatg 600
   agaggcacct tggtttcatg ttaccaagtc atgattactt taggtatttt cttaggttac 660
   tgtaccaatt ttggtaccaa gaattactca aactctgtcc aatggagagt tccattaggt 720
   ttgtgtttcg cctgggcctt atttatgatt ggtggtatga catttgttcc agagtctcca 780
   cgttatttgg ctgaagtcgg taagatcgaa gaagccaaac gttctattgc cgtttataac 840
   aaggttgctg ttgatgatcc atctgttttg gctgaagtcg aagctgtctt ggctggtgta 900
   gaggcagaga aattagctgg taatgcatcc tggggtgaat tgtttagtag caagacaaag 960
   gtccttcagc gtttgatcat gggtgctatg attcaatctc tacaacaatt gacaggtgat 1020
   aactatttct tctactatgg tactactatt ttcaaggctg ttggtttgag tgactctttc 1080
   gaaacctcta ttgtcttggg tattgttaac tttgcttcca cctttgttgg tatttacgtt 1140
   gttgagagat atggtcgtcg tacttgtttg ctatggggtg ctgcatccat gactgcttgt 1200
   atggttgtct atgcttccgt gggtgtcacc agattatggc caaatggtca agaccaacca 1260
   tcttccaagg gtgctggtaa ctgtatgatt gtctttgcct gtttctatat tttctgtttt 1320
   gctactacat gggctccaat tccttatgtc gttgtttctg aaactttccc attgagagtc 1380
   aagtctaagg ctatgtctat tgctacagct gctaattggt tgtggggttt cttgattggt 1440
   ttcttcactc catttattac tggtgctatt aacttctact acggttacgt tttcatgggc 1500
   tgtttggtct tcatgttctt ctatgttttg ttagttgttc cagaaactaa gggtttgact 1560
   ttggaagaag tcaacaccat gtgggaagaa ggtgttctac catggaagtc tgcctcatgg 1620
   gttccaccat ccagaagagg tgccaactac gacgctgaag aaatgactca cgatgacaag 1680
   ccattgtaca agagaatgtt cagcaccaaa taa 1713
<210> 21
   <211> 1693
   <212> DNA
   <213> Artificial/Unknown
<220>
   <221> misc_feature
   <222> () .. ()
   <223> Expression cassette
<400> 21
   tctcgtagga acaatttcgg gcccctgcgt gttcttctga ggttcatctt ttacatttgc 60
   ttctgctgga taattttcag aggcaacaag gaaaaattag atggcaaaaa gtcgtctttc 120
   aaggaaaaat ccccaccatc tttcgagatc ccctgtaact tattggcaac tgaaagaatg 180
   aaaaggagga aaatacaaaa tatactagaa ctgaaaaaaa aaaagtataa atagagacga 240
   tatatgccaa tacttcacaa tgttcgaatc tattcttcat ttgcagctat tgtaaaataa 300
   taaaacatca agaacaaaca agctcaactt gtcttttcta agaacaaaga ataaacacaa 360
   aaacaaaaag tttttttaat tttaatcaaa aaatgtccac aaaatcatat accagtagag 420
   ctgagactca tgcaagtccg gttgcatcga aacttttacg tttaatggat gaaaagaaga 480
   ccaatttgtg tgcttctctt gacgttcgtt cgactgatga gctattgaaa cttgttgaaa 540
   cgttgggtcc atacatttgc cttttgaaaa cacacgttga tatcttggat gatttcagtt 600
   atgagggtac tgtcgttcca ttgaaagcat tggcagagaa atacaagttc ttgatatttg 660
   aggacagaaa attcgccgat atcggtaaca cagtcaaatt acaatataca tcgggcgttt 720
   accgtatcgc agaatggtct gatatcacca acgcccacgg ggttactggt gctggtattg 780
   ttgctggctt gaaacaaggt gcgcaagagg tcaccaaaga accaagggga ttattgatgc 840
   ttgctgaatt gtcttccaag ggttctctag cacacggtga atatactaag ggtaccgttg 900
   atattgcaaa gagtgataaa gatttcgtta ttgggttcat tgctcagaac gatatgggag 960
   gaagagaaga agggtttgat tggctaatca tgaccccagg tgtaggttta gacgacaaag 1020
   gcgatgcatt gggtcagcag tacagaaccg tcgacgaagt tgtaagtggt ggatcagata 1080
   tcatcattgt tggcagagga cttttcgcca agggtagaga tcctaaggtt gaaggtgaaa
   1140
   gatacagaaa tgctggatgg gaagcgtacc aaaagagaat cagcgctccc cattaatttg
   1200
   cgaacacttt tattaattca tgatcacgct ctaatttgtg catttgaaat gtactctaat
   1260
   tctaatttta tatttttaat gatatcttga aaagtaaata cgtttttaat atatacaaaa
   1320
   taatacagtt taattttcaa gtttttgatc atttgttctc agaaagttga gtgggacgga
   1380
   gacaaagaaa ctttaaagag aaatgcaaag tgggaagaag tcagttgttt accgaccgca
   1440
   ctgttattca caaatattcc aattttgcct gcagacccac gtctacaaat tttggttagt
   1500
   ttggtaaatg gtaaggatat agtagagcct ttttgaaatg ggaaatatct tctttttctg
   1560
   tatcccgctt caaaaagtgt ctaatgagtc agttatttct ttcttactca tcgcccgtca
   1620
   cttaaaagaa gaaaaattac tttcatgatg cgaagcgaaa aaaattttta gcttcaattt
   1680
   tcacaatgca tct
   1693